# EUROPÄISCHE PATENTSCHRIFT

(11) **EP 4 568 596 B1**
(45) Veröffentlichungstag und Bekanntmachung des Hinweises auf die Patenterteilung: **26.08.2026**
(21) Anmeldenummer: 23785988.9
(22) Anmeldetag: 29.09.2023
(51) Int. Cl.: A61B 17/29

(54) **KUPPLUNGSVORRICHTUNG UND GRIFFVORRICHTUNG FÜR EIN CHIRURGISCHES INSTRUMENT, CHIRURGISCHES INSTRUMENT SOWIE VERFAHREN ZUM ZUSAMMENBAU UND VERFAHREN ZUR DEMONTAGE DES CHIRURGISCHEN INSTRUMENTS**
COUPLING DEVICE AND HANDLE DEVICE FOR A SURGICAL INSTRUMENT, SURGICAL INSTRUMENT AND METHOD FOR ASSEMBLY AND METHOD FOR DISASSEMBLY OF THE SURGICAL INSTRUMENT
DISPOSITIF DE COUPLAGE ET DISPOSITIF DE POIGNÉE POUR UN INSTRUMENT CHIRURGICAL, INSTRUMENT CHIRURGICAL ET PROCÉDÉ D'ASSEMBLAGE ET PROCÉDÉ DE DÉSASSEMBLAGE DE L'INSTRUMENT CHIRURGICAL

(30) Priorität: 29.09.2022 DE 102022125213
(43) Veröffentlichungstag der Anmeldung: 18.06.2025
(73) Patentinhaber: Karl Storz SE & Co. KG, 78532 Tuttlingen (DE)
(72) Erfinder: SCHNEIDER, Janosz, 78532 Tuttlingen (DE); KÄRCHER, Daniel, 78532 Tuttlingen (DE); MERZ, Robin, 78532 Tuttlingen (DE); SCHNEIDER, Sven, 78532 Tuttlingen (DE); UNGER, Tobias, 78532 Tuttlingen (DE); LÄNGLE, Dominik, 78532 Tuttlingen (DE); PÖNISCH, Judith, 78532 Tuttlingen (DE)
(86) Internationale Anmeldenummer: PCT/EP2023/077019
(87) Internationale Veröffentlichungsnummer: WO 2024/068909

(56) Entgegenhaltungen:
- EP-A1- 2 305 145
- DE-A1- 102009 048 600
- US-A1- 2009 069 842
- US-A1- 2013 172 859

## Beschreibung

Die Erfindung betrifft eine Kupplungsvorrichtung für ein chirurgisches Instrument sowie eine Griffvorrichtung eines chirurgischen Instruments mit einer solchen Kupplungsvorrichtung. Ferner betrifft die Erfindung ein chirurgisches Instrument mit einer solchen Kupplungsvorrichtung selbst sowie ein Verfahren zum Zusammenbau und ein Verfahren zur Demontage des chirurgischen Instruments.

Aus dem Stand der Technik ist bekannt, dass medizinische Instrumente für die endoskopische Chirurgie ein bewegliches Werkzeug an dem distalen Ende eines länglichen Schafts aufweisen, an dessen proximalem Ende eine Betätigungseinheit wie beispielsweise ein Griff mit einem unbeweglichen und einem beweglichen Griffteil zur Betätigung des Werkzeugs angeordnet ist. Dazu weist das chirurgische Instrument ein Kraftübertragungselement auf, für das häufig eine Zug- und Druckstange (abgekürzt Zugstange) eingesetzt wird, und das das Werkzeug mit dem Griff, bzw. dem beweglichen Griffteil in Wirkverbindung bringt. Das Kraftübertragungselement erstreckt sich dazu axial beweglich durch den länglichen Schaft und ist distalseitig mit einer Werkzeugmechanik verbunden, die die längsaxiale Hin- und Herbewegung des Kraftübertragungselements auf das Werkzeug überträgt, beispielsweise zum Öffnen und Schließen von Maulteilen des Werkzeugs, etwa zum Greifen oder Schneiden. Am proximalen Ende steht das Kraftübertragungselement mit dem beweglichen Griffteil in Eingriff, durch den eine Bewegung des beweglichen Griffteils zu dem unbeweglichen Griffteil hin oder davon weg in die längsaxiale Hin- und Herbewegung des Kraftübertragungselements umgesetzt wird.

Ferner ist bekannt, dass modulare Instrumentensysteme existieren, aus denen sich solche medizinischen Instrumente beispielweise aus den Baugruppen Griff, Schaft und Arbeitseinsatz, bestehend aus Kraftübertragungselement und Werkzeug, zusammensetzen lassen, die auch wieder zerlegt, gereinigt und desinfiziert werden können und dadurch zumindest teilweise wiederverwendbar sind. Ein solches modulares chirurgisches Instrumentensystem ist z. B. unter dem Namen Clickline ^{®} Instruments aus dem Katalog "Highlights, Clickline ^{®} Instruments, Laparoscopic Hand Instruments, 01/2020" der Karl Storz GmbH & Co., Tuttlingen, Deutschland, bekannt. Modulare Instrumentensysteme erhöhen zudem die Funktionsvariabilität, indem verschiedene Griffe mit verschiedenen Schäften und/oder Arbeitseinsätzen kombiniert werden können. Dabei können sich zum einen die verschiedenen Griffe infolge unterschiedlicher Hebelverhältnisse hinsichtlich der jeweils erzeugbaren maximalen Kraft unterscheiden, und zum anderen können sich die Kraftübertragungselemente hinsichtlich der mit ihnen maximal übertragbaren Kraft unterscheiden. Die mit dem jeweiligen Kraftübertragungselement maximal übertragbare Kraft kann an die Aufgabe des Werkzeugs am distalen Enden angepasst und durch die Stärke des Kraftübertragungselements bzw. dessen proximale Verbindung mit einem beweglichen Griffteil begrenzt sein.

So können die Kraftübertragungselemente eines zerlegbaren Instrumentensystems bzw. die proximale Verbindung der Kraftübertragungselemente mit einem beweglichen Griffteil in Abhängigkeit der unterschiedlichen Einsatzgebiete und Spezifikationen unterschiedlich dimensioniert sein.

Zur kraftschlüssigen Verbindung solcher Kraftübertragungselemente mit einem beweglichen Griffteil beschreibt EP 2 305 145 B1, die ein solch zerlegbares medizinisches Zangensystem betrifft, Kupplungsvorrichtungen zum Eingriff mit einem Verbindungselement am proximalen Ende des Kraftübertragungselements. Dabei weisen die Verbindungselemente der Kraftübertragungselemente für die unterschiedlichen Einsatzgebiete und Spezifikationen unterschiedliche Querschnitte auf. Ein Verbindungselement kann in einer entsprechend ausgebildeten Aufnahmeausnehmung eines Kupplungselements aufgenommen werden, das mit einem Schieber verbunden ist, der durch das bewegliche Griffteil betätigbar ist. An die erste Aufnahmeausnehmung kann sich in dem Kupplungselement proximalseitig eine weitere Ausnehmung mit kleinerem Durchmesser anschließen, die die Aufnahme eines Verbindungselements mit entsprechend kleinerem Durchmesser gestattet. Zum formschlüssigen Eingriff des jeweiligen Verbindungselements ist eine Drehung des Kupplungselements um die Achse des Kraftübertragungselements erforderlich.

Aus DE 10 2009 048 600 A1 ist ein zerlegbares medizinisches Zangensystem bekannt, bei dem sich das bewegliche Griffteil durch Verschieben gegenüber dem festen Griffteil abnehmen lässt. Dabei wird auch die Zug- / Druckstange entkuppelt, welche das bewegliche Griffteil mit dem Maulteil des Instruments verbindet.

Die Offenlegungsschrift US 2009/0069842 A1 lehrt ein zerlegbares endoskopisches Instrument mit einer Bajonettkupplung.

US 2013/0172859 A1 offenbart ein zerlegbares medizinisches Zangensystem mit einem Kupplungsadapter, der es erlaubt, unterschiedlicher Zugstangen mit r longitudinalen Steck-Kupplungsvorrichtung.

Die formschlüssige Verbindung eines solchen kugelförmigen Verbindungselements des Betätigungselements in einer kugelförmig ausgebildeten Aufnahmeausnehmung des Kupplungselements ist allerdings spielbehaftet. Ferner ist die Kupplungsvorrichtung für höchstens zwei Kugelgrößen ausgelegt, wodurch eine Variabilität in Bezug auf unterschiedliche Werkzeuge reduziert ist, sodass vor allem beim Einsatz distaler Werkzeuge mit geringer Belastbarkeit zusätzliche Kraftbegrenzungsvorrichtungen vorzusehen sind, um das distale Werkzeug vor einer Überlastung durch zu hohe Betätigungskraft des Anwenders am Griff zu schützen.

Ausgehend von diesem Stand der Technik ist es Aufgabe der vorliegenden Erfindung, eine verbesserte Kupplungsvorrichtung für ein chirurgisches Instrument bereitzustellen.

Diese Aufgabe wird durch eine Kupplungsvorrichtung mit den Merkmalen des Anspruchs 1 gelöst.

Die weiteren Aufgaben, eine entsprechend verbesserte Griffvorrichtung für ein chirurgisches Instrument und ein entsprechend verbessertes chirurgisches Instrument bereitzustellen, wird durch eine Griffvorrichtung mit den Merkmalen des Anspruchs 11 und durch ein chirurgisches Instrument mit den Merkmalen des Anspruchs 12 gelöst.

Einen vereinfachten Zusammenbau eines chirurgischen Instruments und dessen Demontage werden durch die Verfahren mit den Merkmalen der unabhängigen Ansprüche 13 und 15 gelöst.

Weiterbildungen sowie bevorzugte Ausführungsformen sind in den jeweiligen Unteransprüchen ausgeführt.

Eine erfindungsgemäße Kupplungsvorrichtung ist gemäß einer ersten Ausführungsform für ein chirurgisches Instrument vorgesehen, das ein Kraftübertragungselement und eine Griffvorrichtung mit einem beweglichen Griffteil aufweist. Hierin werden die Begriffe "proximal" und "distal" zur Lagebezeichnung von Komponenten in Bezug auf einen Benutzer verwendet, der das chirurgische Instrument bedient. Demzufolge steht die Griffvorrichtung mit einem proximalen Ende des Kraftübertragungselements in Verbindung, dessen anderes - distales - Ende zumeist mit einem Werkzeug verbunden ist. Die Kupplungsvorrichtung ist dabei zur Verbindung des beweglichen Griffteils mit dem proximalen Ende des Kraftübertragungselements ausgebildet, um eine durch Benutzerkraft auf das Griffteil aufgebrachte Betätigungsbewegung in eine Längsbewegung des Kraftübertragungselements umzusetzen. Dazu weist das Kraftübertragungselement, das eine Längsachse definiert, die der Längsachse des chirurgischen Instruments entspricht, an seinem proximalen Ende ein abgesetztes Verbindungselement auf. Der Absatz wird durch eine Änderung der Querschnittsabmessungen des Verbindungselements in Bezug auf das Kraftübertragungselement senkrecht zur Längsachse gebildet, wobei die Querschnittsabmessungen des Absatzes kleiner sind als die Querschnittsabmessungen des Verbindungselements. Das bewegliche Griffteil der Griffvorrichtung ist um eine Schwenkachse beweglich, die rechtwinklig zu der Längsachse verläuft, diese aber nicht schneidet. Dabei weist die Kupplungsvorrichtung zur Verbindung des Kraftübertragungselements mit dem beweglichen Griffteil ein Kupplungselement und ein damit zusammenwirkendes Schieberelement auf. In dem Kupplungselement ist eine Ausnehmung zur Aufnahme des Verbindungselements ausgebildet, und das Schieberelement, das in Richtung der Längsachse geführt bewegt werden kann, ist zum Zusammenwirken mit dem beweglichen Griffteil gestaltet.

Erfindungsgemäß ist in dem Schieberelement eine Führungsaufnahme für das Kupplungselement entlang einer Kopplungsachse ausgebildet, die rechtwinklig zu der Längsachse und rechtwinklig zu der Schwenkachse verläuft. Das in der Führungsaufnahme aufgenommene Kupplungselement kann somit entlang der Kopplungsachse bewegt werden. Die Ausnehmung in dem Kupplungselement, die zur Aufnahme des Verbindungselements am proximalen Ende des Kraftübertragungselements vorgesehen ist, verläuft korrespondierend zu der Kopplungsachse, d. h. entlang der Kopplungsachse oder mit einer gewissen Abweichung zu der Kopplungsachse. Dabei kann eine Rotations- bzw. Symmetrieachse der Ausnehmung mit der Kopplungsachse identisch sein, alternativ kann die Rotations- zw. Symmetrieachse der Ausnehmung parallel zu der Kopplungsachse in einer Ebene versetzt sein, die durch die Längs- und Kopplungsachse definiert ist, oder in der Ebene, die durch die Längs- und Kopplungsachse definiert ist, unter einem vorbestimmtem Winkel zu der Kopplungsachse verlaufen. Ferner verjüngt sich der Querschnitt der Ausnehmung in dem Kupplungselement in Richtung der Kopplungsachse - ausgehend von einer Öffnungsseite des Kupplungselements, die von der Führungsaufnahme weg weist. Der Querschnitt der Ausnehmung ist somit an der Öffnungsseite des Kupplungselements am größten und wird mit zunehmendem Abstand von der Öffnungsseite kleiner, wobei sich zumindest die Querschnittsabmessung verringert, die entlang oder parallel zur Längsachse verläuft. Außerdem weist das Kupplungselement distalseitig einen Einschnitt auf, der mit der Ausnehmung verbunden ist. Die Breite des Einschnitts verjüngt sich ebenfalls in einer Richtung parallel zu der Kopplungsachse, wobei die Breite des Einschnitts an der Öffnungsseite des Kupplungselements am größten ist und mit zunehmendem Abstand von der Öffnungsseite kleiner wird.

"Querschnitt" der Ausnehmung bezieht sich hierbei auf eine Fläche senkrecht zur Kopplungsachse. Als "Breite" werden hierin Abmessungen in einer Richtung bezeichnet, die rechtwinklig zur Längsachse und rechtwinkelig zur Kopplungsachse, d. h. parallel zur Schwenkachse liegen.

Das Kupplungselement kann in dem Schieberelement entlang der Kopplungsachse in zumindest einer Freigabeposition, in der das Verbindungselement aus dem Kupplungselement gelöst werden kann, und zumindest einer Eingriffsposition positioniert werden, in der das Verbindungselement mit dem Kupplungselement verbunden ist.

In einer Freigabeposition entlang der Kopplungsachse befindet sich das Kupplungselement, wenn auf einer Höhe der Längsachse die Breite des Einschnitts größer ist als eine Breite des Verbindungselements, sodass das Verbindungselement den Einschnitt nicht hintergreifen kann, sondern bei Bewegung des Kraftübertragungselements in Richtung der Längsachse durch ein Einschnitt in die Ausnehmung eingeführt und daraus herausgezogen werden kann. Als Freigabeposition des Kupplungselements wird auch eine Positionierung verstanden, bei der das Kupplungselement soweit entlang der Kupplungsachse von der Längsachse wegbewegt wird, bis sich das Verbindungselement außerhalb der Ausnehmung befindet. Da sich die Einschnittbreite außerhalb der Ausnehmung quasi um den gesamten Umfang der Ausnehmung erstreckt, kann in einem solchen Fall die Breite des Einschnitts an der Öffnungsseite der Ausnehmung kleiner sein als die Breite des Verbindungselements.

Eine Eingriffsposition entlang der Kopplungsachse wird durch das Kupplungselement eingenommen, wenn auf der Höhe der Längsachse die Querschnittsabmessung der Ausnehmung in Richtung der Längsachse mit einer Längsschnittabmessung des Verbindungselements korrespondiert und zudem die Breite des Einschnitts kleiner ist als die Breite des Verbindungselements, wobei die Breite des Einschnitts zumindest einer Breite des Absatzes entspricht, mit dem das Verbindungselement von dem Kraftübertragungselement abgesetzt ist. Dann hintergreift das Verbindungselement den Einschnitt und kommt in der Ausnehmung zur Anlage. Dabei liegt das Verbindungselement mit zumindest an einem dem Einschnitt gegenüberliegenden proximalen Abschnitt der Innenwandung an und kontaktiert beidseitig die distalseitigen Innenwandabschnitte, die den Einschnitt begrenzen. Auf diese Weise ist das Kraftübertragungselement in Längsrichtung spielfrei mit der Kupplungsvorrichtung verbunden. In einem chirurgischen Instrument ist damit eine exakte Längsbewegung des Kraftübertragungselements durch das Griffteil zur Betätigung eines distalen Werkzeugs möglich, dessen Funktionssicherheit damit verbessert ist. Ferner vereinfacht die erfindungsgemäße Kupplungsvorrichtung die Zerlegung und den Zusammenbau eines chirurgischen Instruments durch das entlang der Kopplungsachse bewegbare Kopplungselement.

Die erfindungsgemäße Kupplungsvorrichtung ist vorteilhaft einsetzbar in einem zerlegbaren chirurgischen Instrument, das aus einem modularen Instrumentensystem zusammensetzbar ist. Dabei stellt eben die Kupplungsvorrichtung die Kraftübertragung von einem beweglichen Griffteil einer Griffvorrichtung auf ein mit einem distalen Werkzeug verbundenen Kraftübertragungselement bereit, wobei das modulare Instrumentensystem unterschiedliche Schäfte, unterschiedliche Griffvorrichtungen und unterschiedliche Werkzeuge mit unterschiedlichen, an eine jeweilige Grenzkraft der unterschiedlichen Werkzeuge angepassten Kraftübertragungselementen aufweisen kann, die nach Bedarf vorteilhaft zu einem chirurgischen Instrument kombiniert werden können.

Vorteilhaft stellt die erfindungsgemäße Kupplungsvorrichtung also gegenüber dem Stand der Technik eine Reduzierung des Spiels bei der Kopplung des jeweiligen Kraftübertragungselements bzw. dessen Verbindungselements mit einem bewegbaren Griffteil der Griffvorrichtung bereit und erhöht die Funktionssicherheit des distalseitig angeordneten Werkzeugs. Ferner gestattet die erfindungsgemäße Kupplungsvorrichtung nicht nur den Eingriff mit einer Vielzahl unterschiedlicher Kraftübertragungselemente, die an eine für ein jeweiliges Werkzeug vorbestimmte Grenzkraft angepasst sind, sondern stellt selbst eine gewisse Anpassung des Kraftübertragungsverhältnisses zwischen einer Betätigungskraft, die durch einen Anwender am beweglichen Griffteil aufgebracht wird, und der Kraft, die mit der Bewegung des Kraftübertragungselement auf das Werkzeug übertragen wird, bereit.

Zwischen der Freigabeposition und der Eingriffsposition des Kupplungselements können Nichteingriffs- oder Übergangspositionen liegen, in denen das Verbindungselement nicht spielfrei in der Ausnehmung des Kupplungselements aufgenommen ist. Dies ist der Fall, wenn eine Querschnittsabmessung der Ausnehmung auf Höhe und in Richtung der Längsachse größer ist als eine Längsschnittfläche des Verbindungselements, und die Breite des Einschnitts auf der Höhe der Längsachse noch kleiner ist als die Breite des Verbindungselements. Dann besteht kein spielfreier Eingriff des Verbindungselements mit dem Kopplungselements, obwohl das Verbindungselement nicht aus dem Kupplungselement gelöst werden kann.

Wenn die Ausnehmung im Kupplungselement einen kreisförmigen Querschnitt hat und das Verbindungselement kugelförmig ist, werden die "Querschnittsabmessung der Ausnehmung" und die "Längsschnittabmessung des Verbindungselements" als der jeweilige Durchmesser der Ausnehmung und des Verbindungselements definiert.

Ansonsten, d. h., wenn die Ausnehmung im Kupplungselement einen polygonalen Querschnitt aufweist, bezieht sich die "Querschnittsabmessung auf Höhe und in Richtung der Längsachse" auf den Abstand einer proximalseitigen Innenwand der Ausnehmung gegenüber dem Einschnitt von der Einschnittöffnung, bzw. von den distalseitigen Innenwandabschnitten, die den Einschnitt begrenzen und einen Anschlag für den Hintergriff des Verbindungselements bereitstellen.

Die "Längsschnittabmessung des Verbindungselements" ist die Abmessung des Verbindungselements in Richtung der Längsachse zwischen proximalen und distalen Kontaktstellen bzw. - flächen, die in der Eingriffsposition an den gegenüberliegenden proximalen und distalen Innenwandabschnitten des Kupplungselements zur Anlage kommen.

Um die spielfreie Aufnahme des Verbindungselements in der Ausnehmung des Kupplungselements weiter zu verbessern, ist gemäß einer weiteren Ausführungsform der erfindungsgemäßen Kupplungsvorrichtung vorgesehen, dass eine Querschnittsform der Ausnehmung im Kupplungselement senkrecht zur Kupplungsachse mit einer Form einer Längsschnittfläche des Verbindungselements senkrecht zur Kupplungsachse korrespondiert.

Somit können nach einer bevorzugten Ausführungsform der erfindungsgemäßen Kupplungsvorrichtung das Verbindungselement am proximalen Ende des Kraftübertragungselements kugelförmig und die Ausnehmung im Kupplungselement kegel- oder kegelstumpfförmigen mit einem kreisförmigen Querschnitt ausgebildet sein. Die Öffnungsseite der Ausnehmung entspricht dann einer Grundfläche der Kegelform bzw. Kegelstumpfform, die den größten Durchmesser der Ausnehmung aufweist. Die Kegelspitze der Kegelform bzw. Deckfläche der Kegelstumpfform weist den kleinsten Durchmesser der Ausnehmung auf. Um Aufnahme in der Ausnehmung finden zu können, liegt der Durchmesser des kugelförmigen Verbindungselements liegt zwischen dem größten und kleinsten Durchmesser der Ausnehmung. Als Variation der Kugelform des Verbindungselements sollen hierbei auch Ellipsoide und Ovoide umfasst sein, für die die kegelförmige bzw. kegelstumpfförmige Ausnehmung mit einer entsprechenden elliptischen oder ovalen Querschnittsform ausgebildet sein kann, sodass die Längsschnittabmessung des Verbindungselements bzw. Querschnittabmessung der Ausnehmung in Längsrichtung der Hauptachse des elliptischen bzw. Symmetrieachse des ovalen Querschnitts entsprechen.

Grundsätzlich ist es aber auch möglich, dass die Querschnittsform der Ausnehmung im Kupplungselement senkrecht zur Kupplungsachse von der Form der Längsschnittfläche des Verbindungselements senkrecht zur Kupplungsachse abweicht: Beispielsweise kann ein kugelförmiges Kupplungselement auch in einer Ausnehmung mit polygonaler Querschnittsform Aufnahme finden. Die sich verjüngende Ausnehmung kann eine Pyramiden- oder Pyramidenstumpfform haben, oder aber ein dreieckiges oder trapezförmiges Verjüngungsprofil aufweisen, bei dem die proximale und/oder distale Innenwandabschnitte in Bezug zur Kupplungsachse aufeinander zu verlaufen.

Bezüglich des Verlaufs der sich verjüngenden Ausnehmung in dem Kupplungselement entlang der Kopplungsachse kann zwar aufgrund der einfacheren Fertigung ein gerader Verlauf in Bezug auf die Kupplungsachse bevorzugt sein, sodass z. B. bei einer kegelförmigen oder kegelstumpfförmigen Ausnehmung die Kupplungsachse des Kupplungselements der Rotationsachse der Ausnehmung entspricht. Allerdings ist es auch möglich, dass die sich verjüngende Ausnehmung in dem Kupplungselement entlang der Kopplungsachse schief verläuft, sodass z. B. die Rotationsachse der schief kegelförmigen oder kegelstumpfförmigen Ausnehmung nicht der Kupplungsachse des Kupplungselements entspricht. Außerdem kann zwar eine stetiger und konstanter Verjüngungsverlauf der Ausnehmung bevorzugt sein, der Querschnitt der Ausnehmung kann sich aber auch abschnittsweise entlang der Kopplungsachse und/oder mit unterschiedlichen Neigungen verjüngen. Entsprechendes gilt für den Einschnitt.

Da die sich verjüngende Ausnehmung des Kupplungselements unterschiedliche Querschnittsabmessungen und der sich entsprechend verjüngende Einschnitt unterschiedliche Breiten aufweisen, können verschiedene Kraftübertragungselemente, deren Verbindungselemente unterschiedliche Längsabmessungen und Breiten aufweisen, durch die erfindungsgemäße Kopplungsvorrichtung in einfacher Weise spielfrei mit dem beweglichen Griffteil verbunden werden. Dazu muss lediglich das Kopplungselement entlang der Kopplungsachse in eine jeweilige Eingriffsposition bewegt werden, in der die Ausnehmung eine passende Querschnittabmessung in Längsrichtung und der Einschnitt eine geeignete Breite für das jeweilige Verbindungselement aufweist.

Entsprechend kann die erfindungsgemäße Kupplungsvorrichtung gemäß einer weiteren Ausführungsform wahlweise mit einem ersten Kraftübertragungselement oder mit einem zweiten Kraftübertragungselement (oder mit weiteren Kraftübertragungselementen) in Eingriff gebracht werden, die jeweils ein durch einen Absatz abgesetztes Verbindungselement aufweisen. Das erste Kraftübertragungselement, das zur Übertragung einer ersten Grenzkraft ausgebildet ist, weist ein erstes Verbindungselement auf, und das zweite Kraftübertragungselement, das zur Übertragung einer zweiten Grenzkraft ausgebildet ist, die größer ist als die erste Grenzkraft, weist ein zweites Verbindungselement auf. Dabei sind die Längsschnittabmessung und die Breits des zweiten Verbindungselements größer als die Längsschnittabmessung und die Breite des ersten Verbindungselements. Außerdem kann die Absatzbreite des zweiten Kraftübertragungselements größer sein als die Absatzbreite des ersten Kraftübertragungselements. Entsprechendes gilt für weitere Kraftübertragungselemente, die zur Übertragung einer weiteren Grenzkraft mit einem weiteren Verbindungselement ausgebildet sind, dessen Längsschnittabmessung und Breite in Abhängigkeit der weiteren Grenzkraft ausgebildet sind.

Dabei kann das Kupplungselement entlang der Kopplungsachse zum Eingriff mit dem ersten Kraftübertragungselement in einer ersten Eingriffsposition und zum Eingriff mit dem zweiten Kraftübertragungselement in einer zweiten Eingriffsposition angeordnet werden, die sich von der ersten Eingriffsposition unterscheidet.

In der ersten Eingriffsposition korrespondiert die Querschnittsabmessung der Ausnehmung auf der Höhe und in Richtung der Längsachse mit der Längsschnittabmessung des ersten Verbindungselements und die Breite des Einschnitts ist kleiner als die Breite des ersten Verbindungselements, sodass das erste Verbindungselement den Einschnitt hintergreift und in der Ausnehmung zur Anlage kommt. In der zweiten Eingriffsposition korrespondiert die Querschnittsabmessung der Ausnehmung auf der Höhe und in Richtung der Längsachse mit der Längsschnittabmessung des zweiten Verbindungselements und die Breite des Einschnitts ist kleiner als die Breite des ersten Verbindungselements, sodass das zweite Verbindungselement den Einschnitt hintergreift und in der Ausnehmung zur Anlage kommt. Da das zweite Verbindungselement größer ist als das erste Verbindungselement, liegt das zweite Verbindungselement in Eingriffslage an einem Ausnehmungsquerschnitt an, der näher an der Öffnungsseite der Ausnehmung ist, als der Ausnehmungsquerschnitt, an dem das zweite Verbindungselement in Eingriffslage anliegt. Folglich ist das entlang der Kopplungsachse relativ zu Längsachse bewegbare Kupplungselement in der zweiten Eingriffsposition bei Eingriff mit dem zweiten Kraftübertragungselement weiter in das Schieberelement aufgenommen, als in der ersten Eingriffsposition.

Nach einer weiteren Ausführungsform kann die erfindungsgemäße Kupplungsvorrichtung eine Lagerkomponente aufweisen, die in einem Gehäuse der Griffvorrichtung vorliegt, d. h. darin angeordnet bzw. damit verbunden oder ggf. einstückig damit ausgebildet ist. Die Lagerkomponente weist zwei Führungsschienen auf, die sich parallel zu der Längsachse zur Führung des Schieberelements in Längsrichtung erstrecken. Das Schieberelement weist auf einer von seiner Führungsaufnahme abgewandten Seite ein Führungsprofil mit einem entlang der Kopplungsachse ausgebildeten Führungszapfen auf, der zur Aufnahme zwischen den beiden Führungsschienen ausgebildet ist.

Um den Führungsweg des Schieberelements zu begrenzen, kann nach einer weiteren Ausführungsform jede Führungsschiene einen Führungsabschnitt aufweisen, der in Längsrichtung beidseitig durch jeweils einen Anschlag begrenzt ist. Entsprechend kann das Schieberelement benachbart zu dem Führungszapfen beidseitig jeweils einen Auflagerabschnitt aufweisen, der auf dem Führungsabschnitt gleitend gelagert ist.

Ferner kann eine erfindungsgemäße Kupplungsvorrichtung nach einer weiteren Ausführungsform zumindest einen an der Lagerkomponente um die Schwenkachse schwenkbar gelagerten Schwenkbügel aufweisen, um das Schieberelement mit dem beweglichen Griffteil zu verbinden. In einer bevorzugten Ausführungsform können zwei parallel orientierte, beidseitig an der Lagerkomponente schwenkbar gelagerte Schwenkbügel zur Verbindung des Schieberelements mit dem beweglichen Griffteil zur gleichmäßig verteilten Kraftübertragung vorgesehen sein. Dazu kann ein Schwenkbügel einen Mitnehmerabschnitt für das Schieberelement und einen Verbindungsabschnitt aufweisen, der zur Verbindung mit dem beweglichen Griffteil vorgesehen ist, wobei die Schwenkachse durch einen Zentralabschnitt zwischen dem Mitnehmerabschnitt und dem Verbindungsabschnitt verläuft. Dabei weist der bzw. jeder Schwenkbügel - im Zentralabschnitt - zur Bereitstellung der Schwenkachse eine koaxiale Schwenklagervorrichtung auf, beispielsweise eine Schwenklageröffnung oder ein Schwenklager-Achsstummel. Die Lagerkomponente hat an zumindest einer der Führungsschienen, bevorzugt an beiden Führungsschienen, eine zu der Schwenkachse koaxiale Lagervorrichtung, die zur Zusammenwirkung mit der Schwenklagervorrichtung zur schwenkbaren Lagerung des Schwenkbügels ausgebildet ist. Entsprechend kann die Lagervorrichtung beispielsweise zum Zusammenwirken mit einer Schwenklageröffnung ein Achsstummel oder zum Zusammenwirken mit einem Schwenklager-Achsstummel eine Lageröffnung sein. Ferner können sowohl Schwenklagervorrichtung als auch Lagervorrichtung als Öffnungen ausgebildet sein, wobei das Zusammenwirken der Schwenklageröffnung und der Lageröffnung durch ein separates Achselement bereitgestellt wird, das in den Öffnungen aufgenommen wird.

Nach einer noch weiteren Ausführungsform der erfindungsgemäßen Kupplungsvorrichtung kann vorgesehen sein, dass das Schieberelement distalseitig eine Durchtrittsöffnung zum Durchtritt des Kraftübertragungselements aufweist. Die Durchtrittsöffnung ist mit der in dem Schieberelement ausgebildeten Führungsaufnahme verbunden und überlagert den Einschnitt des in dem Schieberelement aufgenommenen Kupplungselements. Dabei weist das Kupplungselement auf einer zur Schwenkachse parallelen Achse zumindest einen nach außen ragenden Führungsstift, bevorzugt zwei nach außen ragende Führungsstifte, auf. Und in dem Schieberelement ist zumindest ein Führungsspalt bzw. sind bevorzugt zwei Führungsspalte ausgebildet, der/die parallel zur Kopplungsachse verläuft/verlaufen und mit der Führungsaufnahme verbunden ist bzw. sind. Dabei ist der Führungsstift bzw. sind die Führungsstifte in dem Führungsspalt bzw. in den Führungsspalten angeordnet.

Nach einer noch weiteren Ausführungsform der erfindungsgemäßen Kupplungsvorrichtung ist der Führungsstift bzw. sind die Führungsstifte zum Zusammenwirken mit dem Schwenkbügel bzw. den Schwenkbügeln ausgebildet. Zu diesem Zweck ragt der bzw. jeder Führungsstift mit einem Endabschnitt aus dem Führungsspalt des Schieberelements heraus. Der bzw. jeder Schwenkbügel weist in dem Mitnehmerabschnitt eine Führungsnut auf, die in radialer Richtung zu der Schwenklagervorrichtung verläuft und in der der Endabschnitt des Führungsstifts aufgenommen ist. Somit wird das Verschwenken des Schwenkbügels bei Betätigung des Griffteils über den in der Führungsnut des Schwenkbügels und dem Führungsspalt des Schieberelements geführten Führungsstift in eine Längsbewegung des Schieberelements entlang der Führungsschienen übersetzt. Entsprechend wird das mit dem Schieberelement über das Kupplungselement verbundene Kraftübertragungselement ebenfalls in Längsrichtung bewegt.

Dabei variiert die Position der parallel zur Schwenkachse verlaufenden Führungsstift-Achse mit der Eingriffsposition des Kupplungselements und ist demzufolge von den Abmessungen des Verbindungselements des jeweils eingesetzten Kraftübertragungselements abhängig, während die Position der Schwenkachse an der Lagerkomponente festgelegt ist. Vorteilhaft gestattet das hierdurch bedingte variable Hebelverhältnis eine gewisse Anpassung des Kräfteverhältnisses von einer an dem beweglichen Griffteil aufgebrachten Betätigungskraft zu einer axialen Bewegungskraft des Kraftübertragungselements. Das Hebelverhältnis besteht zwischen einem ersten, konstanten Hebel, der zwischen der Schwenkachse und dem Griffteil definiert wird, an dem die Betätigungskraft wirkt, und einem zweiten, variablen Hebel, der durch den Abstand der Schwenkachse von der Führungsstift-Achse bei in Eingriffsposition befindlichem Kupplungselement definiert ist. Der zweite Hebel ist damit von den Abmessungen des Verbindungselements abhängig, das mit dem Kupplungselement in Eingriffsposition verbunden ist. Mit der Variation des Hebelverhältnisses ist eine entsprechende Variation des Kraftübertragungsverhältnisses verbunden, die zum Schutz weiterer Instrumentenbauteile wie einem distalen Werkzeug beiträgt. So wird das Hebelverhältnis bei Eingriff kleinerer Verbindungselemente mit dem Kupplungselement kleiner, da das Kupplungselement für die Eingriffsposition entlang der Kupplungsachse weiter aus dem Schieberelement herausragt und damit der zweite Hebel, d. h. der Abstand der Führungsstift-Achse zur Schwenkachse, größer wird. Entsprechend dem Hebelverhältnis wird bei gleicher Betätigungskraft am Griffteil die Kraft zur Längsbewegung des Kraftübertragungselements verringert.

Nach einer weiteren Ausführungsform der erfindungsgemäßen Kupplungsvorrichtung weist diese Federelement auf, das sich an einem Boden der Führungsaufnahme des Schieberelements abstützt und eine Kraft in Richtung der Kopplungsachse auf das Kupplungselement ausübt, um das Kupplungselement in der Eingriffsposition zu halten. Das Federelement kann beispielsweise eine mechanische Druck- bzw. Schraubenfeder sein, oder als pneumatisches, hydraulisches oder magnetisches Federelement ausgebildet sein.

Um die Anordnung des Federelements zu erleichtern, wird nach einer noch weiteren Ausführungsform der erfindungsgemäßen Kupplungsvorrichtung vorgeschlagen, dass das Schieberelement eine Aufnahmeausnehmung für das Federelement aufweist. Die Aufnahmeausnehmung schließt sich unter Ausbildung eines ringförmigen Absatzes an die Führungsaufnahme an. Ferner weist die Aufnahmeausnehmung, die entsprechend zur Anordnung zw. Aufnahme des Federelements dimensioniert und in Richtung Kopplungsachse ausgebildet ist, dann den Boden auf, an dem sich das Federelement abstützt. Alternativ oder zusätzlich dazu kann das Kupplungselement einen von der Öffnungsseite der Ausnehmung an der Oberseite des Kupplungselements abgewandten Stutzen aufweisen, der für einen definierten Eingriff mit dem Federelement sorgt. So kann der Stutzen koaxial zur Kopplungsachse unter Bildung eines ringförmigen Anschlags an der Unterseite des Kupplungselements ausgebildet sein. Dabei ist der Stutzen so dimensioniert , dass er zumindest teilweise mit dem Federelement in die Aufnahmeausnehmung aufgenommen werden kann, wenn dies für eine entsprechende Anordnung des Kupplungselements entlang der Kupplungsachse in einer Eingriffs- oder Freigabeposition erforderlich ist.

Nach einer weiteren Ausführungsform der erfindungsgemäßen Kupplungsvorrichtung wird vorgeschlagen, dass die Lagerkomponente einen Blockabschnitt aufweist, an dem die Führungsschienen angeordnet sind, die sich parallel zu der Längsachse und proximalseitig von dem Blockabschnitt weg erstrecken. Der Blockabschnitt kann dabei den distalen Anschlag des Führungsabschnitts jeder Führungsschiene bereitstellen. Durch den Blockabschnitt erstreckt sich eine Durchgangsöffnung für das Kraftübertragungselement entlang der Längsachse, sodass sich die Durchgangsöffnung im Blockabschnitt und die Durchtrittsöffnung im Schieberelement überlagern.

Eine erfindungsgemäße Griffvorrichtung für ein chirurgisches Instrument ist nach einer ersten Ausführungsform zur Anordnung an einem proximalen Ende eines Schafts vorgesehen, durch den sich ein Kraftübertragungselement erstreckt, das eine Längsachse definiert und an einem proximalen Ende ein abgesetztes Verbindungselement aufweist. Die Griffvorrichtung weist ein um eine Schwenkachse bewegliches Griffteil und eine erfindungsgemäße Kupplungsvorrichtung zur Verbindung des beweglichen Griffteils mit dem Kraftübertragungselement auf.

Dabei kann das mit der erfindungsgemäßen Kupplungsvorrichtung ausgestattete zerlegbare chirurgische Instrument, aus einem modularen Instrumentensystem zusammengesetzt sein, bei dem die Kupplungsvorrichtung die Kraftübertragung von dem beweglichen Griffteil einer erfindungsgemäßen Griffvorrichtung auf ein mit einem distalen Werkzeug verbundenen Kraftübertragungselement bereitstellt. Das modulare Instrumentensystem kann, wie ausgeführt, unterschiedliche Schäfte, unterschiedliche Griffvorrichtungen und unterschiedliche Werkzeuge mit unterschiedlichen, an eine jeweilige Grenzkraft der unterschiedlichen Werkzeuge angepassten Kraftübertragungselementen aufweisen, die nach Bedarf zu einem chirurgischen Instrument kombiniert werden können.

Ein erfindungsgemäßes chirurgisches Instrument weist nach einer ersten Ausführungsform einen Schaft auf, durch den sich ein entlang einer Längsachse bewegbares Kraftübertragungselement mit einem abgesetzten Verbindungselement an einem proximalen Ende erstreckt. Das chirurgische Instrument weist an einem proximalen Ende des Schafts eine Griffvorrichtung mit einem Griffteil, das um eine senkrecht zu der Längsachse verlaufenden Schwenkachse beweglich ist, und an einem distalen Ende des Schafts ein Werkzeug auf, das mit dem Kraftübertragungselement in Wirkverbindung steht. Ferner weist das chirurgische Instrument eine erfindungsgemäße Kupplungsvorrichtung zur Verbindung des beweglichen Griffteils mit dem Kraftübertragungselement auf.

Ein erfindungsgemäßes Verfahren zum Zusammenbau eines chirurgischen Instruments mit einer Griffvorrichtung und einem Kraftübertragungselement mit einem abgesetzten Verbindungselement an einem proximalen Ende wird unter Verwendung einer erfindungsgemäßen Kupplungsvorrichtung ausgeführt. Gemäß einer ersten Ausführungsform umfasst das erfindungsgemäße Verfahren die folgenden Schritte:
- Anordnen des Kupplungselements in der Führungsaufnahme des Schieberelements entlang der Kopplungsachse in der Freigabeposition, in der auf der Höhe der Längsachse die Breite des Einschnitts größer ist als die Breite des Verbindungselements;
- Einführen des Verbindungselements am proximalen Ende des Kraftübertragungselements entlang der Längsachse in die Kupplungsvorrichtung durch den Einschnitt in die Ausnehmung; (im Grunde bis das Verbindungselement durch den Einschnitt in die Ausnehmung gelangt und die Kopplungsachse erreicht bzw. kreuzt),
- Überführen des Kupplungselements in die Eingriffsposition, in der auf der Höhe der Längsachse die Querschnittsabmessung der Ausnehmung in Richtung der Längsachse mit der Längsschnittabmessung des Verbindungselements korrespondiert und die Breite des Einschnitts kleiner ist als die Breite des Verbindungselements, sodass das Verbindungselement den Einschnitt hintergreift und in der Ausnehmung zur Anlage kommt.

Das verfahrensgemäß zusammenbaubare chirurgische Instrument kann dabei aus einem modularen Instrumentensystem zusammensetzbar sein.

Nach einer weiteren Ausführungsform des erfindungsgemäßen Verfahrens zum Zusammenbau eines chirurgischen Instruments wird vorgeschlagen, dass das Kraftübertragungselement, das zur Übertragung einer vorbestimmten Grenzkraft ausgebildet ist, entsprechend einer für ein Werkzeug vorbestimmten maximalen Grenzkraft ausgewählt wird, das an einem distalen Ende des Kraftübertragungselements angeordnet wird. Dabei umfasst der Zusammenbau eines chirurgischen Instruments daher auch, dass das Werkzeug mit dem ausgewählten Kraftübertragungselement verbunden wird, das an eine für das Werkzeug vorbestimmte Grenzkraft angepasst ist. Weitere Schritte beim Zusammenbau umfassen, dass das Kraftübertragungselement mit dem proximalseitigen Verbindungselement durch einen Schaft bis in die Griffvorrichtung eingeführt wird, an dem distalseitig das Werkzeug und proximalseitig die Griffvorrichtung angeordnet wird.

Ein ebenfalls erfindungsgemäßes Verfahren zur Demontage eines chirurgisches Instruments mit einer Griffvorrichtung und einem Kraftübertragungselement mit einem abgesetzten Verbindungselement an einem proximalen Ende erfolgt gemäß einer ersten Ausführungsform unter Verwendung einer erfindungsgemäßen Kupplungsvorrichtung und umfasst die folgenden Schritte:
- Überführen des Kupplungselements aus der Eingriffsposition, in der auf der Höhe der Längsachse die Querschnittsabmessung der Ausnehmung in Richtung der Längsachse mit der Längsschnittabmessung des Verbindungselements korrespondiert und die Breite des Einschnitts kleiner ist als die Breite des Verbindungselements, in die Freigabeposition, in der auf der Höhe der Längsachse die Breite des Einschnitts größer ist als die Breite des Verbindungselements; und
- Entfernen des Verbindungselements aus der Ausnehmung des Kupplungselements durch den Einschnitt durch Herausziehen des Kraftübertragungselements entlang der Längsachse.

Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung. Die Zeichnungen, die Beschreibung und die Ansprüche enthalten zahlreiche Merkmale in Kombination. Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Dabei zeigen:
- Fig. 1: eine teilgeschnittene Seitenansicht eines erfindungsgemäßen chirurgischen Instruments mit einer schematisch angedeuteten erfindungsgemäßen Kupplungsvorrichtung,
- Fig. 2: eine Seitenansicht einer erfindungsgemäßen Griffvorrichtung für ein chirurgisches Instrument mit einer schematisch angedeuteten erfindungsgemäßen Kupplungsvorrichtung,
- Fig. 3: eine perspektivische Ansicht einer erfindungsgemäßen Kupplungsvorrichtung,
- Fig. 4: eine Draufsicht auf die Kupplungsvorrichtung aus Fig. 3 ohne Kraftübertragungselement,
- Fig. 5: eine Explosionsansicht der Kupplungsvorrichtung aus Fig. 3,
- Fig. 6: eine Längsschnittansicht durch die Kupplungsvorrichtung entlang Schnittlinie AA aus Fig. 4 mit einem ersten Kraftübertragungselement,
- Fig. 7: eine Längsschnittansicht durch die Kupplungsvorrichtung entlang Schnittlinie AA aus Fig. 4 mit einem zweiten Kraftübertragungselement,
- Fig. 8: eine Längsschnittansicht entsprechend Fig. 6 mit betätigtem ersten Kraftübertragungselement,
- Fig. 9: eine Längsschnittansicht entsprechend Fig. 7 mit betätigtem zweiten Kraftübertragungselement,
- Fig. 10: eine Seitenansicht der Kupplungsvorrichtung aus Fig. 3 in einer ersten Eingriffsposition,
- Fig. 11: eine Seitenansicht der Kupplungsvorrichtung aus Fig. 3 in einer zweiten Eingriffsposition,
- Fig. 12: eine Querschnittansicht durch die Kupplungsvorrichtung entlang Schnittlinie BB aus Fig. 10,
- Fig. 13: eine Querschnittansicht entsprechend Fig. 12 mit Kraftübertragungselement,
- Fig. 14: eine Vorderansicht auf das teilgeschnittene Schieberelement mit Kupplungselement und Schwenkbügeln der Kupplungsvorrichtung aus Fig. 3,
- Fig. 15: eine Schnittansicht in einer Ebene senkrecht zur Kopplungsachse durch ein in der Ausnehmung eines Kupplungselements befindlichen Verbindungselement gemäß einer bevorzugten Ausführungsform in a) Eingriffsposition, b) Übergangsposition und c) Freigabeposition,
- Fig. 16: eine Schnittansicht in einer Ebene senkrecht zur Kopplungsachse durch ein in der Ausnehmung eines Kupplungselements befindlichen Verbindungselement gemäß einer alternativen Ausführungsform in a) Eingriffsposition und b) Freigabeposition.

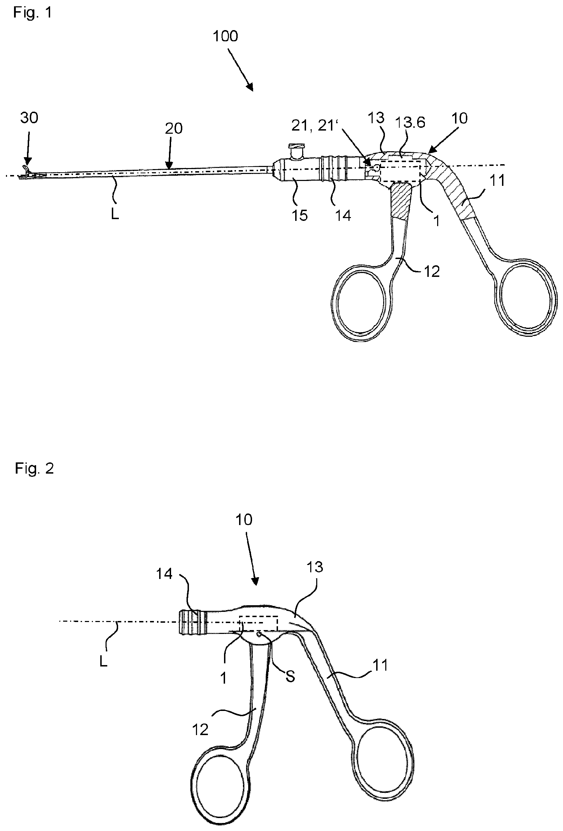

Fig. 1 zeigt ein erfindungsgemäßes zerlegbares chirurgisches Instrument 100, das aus einer Griffvorrichtung 10 mit einer erfindungsgemäßen Kupplungsvorrichtung 1 (dort nur als gestrichelter Kasten dargestellt), einem Schaft 20 und einem Werkzeug 30 mit einem Kraftübertragungselement 21, 21' zusammengesetzt ist. Die Griffvorrichtung 10, die auch in Fig. 2 dargestellt ist, ist mit einem proximalen Endabschnitt 15 des Schafts 20 verbunden. Dazu weist die dort gezeigte Griffvorrichtung 10 einen Verbindungsabschnitt 14 auf, der an einem Gehäuse 13 der Griffvorrichtung 10 ausgebildet ist. In diesem Gehäuse 13 befindet sich ein Aufnahmeraum 13.6 für die Kupplungsvorrichtung 1, die einerseits mit einem in dem Gehäuse 13 um eine Schwenkachse S schwenkbar gelagerten Griffteil 12 in Wirkverbindung steht, das relativ zu einem mit dem Gehäuse 13 verbundenen, unbeweglichen Griffteil 11 bewegt werden kann.

Andererseits steht die Kupplungsvorrichtung 1 mit dem Kraftübertragungselement 21, 21' in Eingriff, das sich durch den Schaft 20 entlang der Längsachse L zu dem Werkzeug 30 erstreckt, das am distalen Ende des Schafts 20 angeordnet ist. So wird eine Betätigung der Griffvorrichtung 10 an den Griffteilen 11, 12 durch die Kupplungsvorrichtung 1 in eine Bewegung des Kraftübertragungselements 21, 21' entlang der Längsachse L umgesetzt. Die Kupplungsvorrichtung 1 sorgt dabei ferner dafür, dass das Kräfteverhältnis der auf die Griffteile aufgebrachten Betätigungskraft zu der mit dem Kraftübertragungselement übertragenen Kraft angepasst werden kann.

Mit der Längsbewegung des Kraftübertragungselements 21, 21' bewirkt die dadurch übertragene Kraft die Betätigung des Werkzeugs 30. In dem in Fig. 1 und 2 dargestellten Beispiel ist das den Aufnahmeraum 13.6 begrenzende Gehäuse 13 mit dem Verbindungsabschnitt 14 einstückig mit dem unbeweglichen Griffteil 11 ausgeführt. Nicht dargestellt sind alternative Ausführungsformen von erfindungsgemäßen Griffvorrichtungen 10, die aus zwei oder mehr Gehäusekomponenten zusammengesetzt sind, die zusammengesetzt der dargestellten einstückigen Gehäusekomponente 13 entsprechen können. Weitere Griffvorrichtungen können sich von dem dargestellten Beispiel hinsichtlich des Verbindungsabschnitts unterscheiden, an dem die Griffvorrichtung mit dem Schaft verbunden wird. Dieser wiederum kann auf ohne einen derartigen proximalen Endabschnitt 15 oder durch einen alternativ gestalteten proximalen Endabschnitt zur Verbindung mit einer Griffvorrichtung ausgebildet sein.

Das in Fig. 1 dargestellte Werkzeug 30 ist ein Greifwerkzeug mit zwei Maulteilen, das eine hier nicht näher erläuterte Mechanik aufweist, über die das Werkzeug 30 mit dem Kraftübertragungselement 21, 21' verbunden ist und die dessen Längsbewegung in Werkzeugbewegungen umsetzt. Vorliegend sind diese Werkzeugbewegungen Öffnungs- und Schließbewegungen der Maulteile. Selbstverständlich können auch andere Werkzeuge mit abweichenden Funktionen, die mit einem Kraftübertragungselement verbunden werden, in einem erfindungsgemäßen chirurgischen Instrument eingesetzt werden.

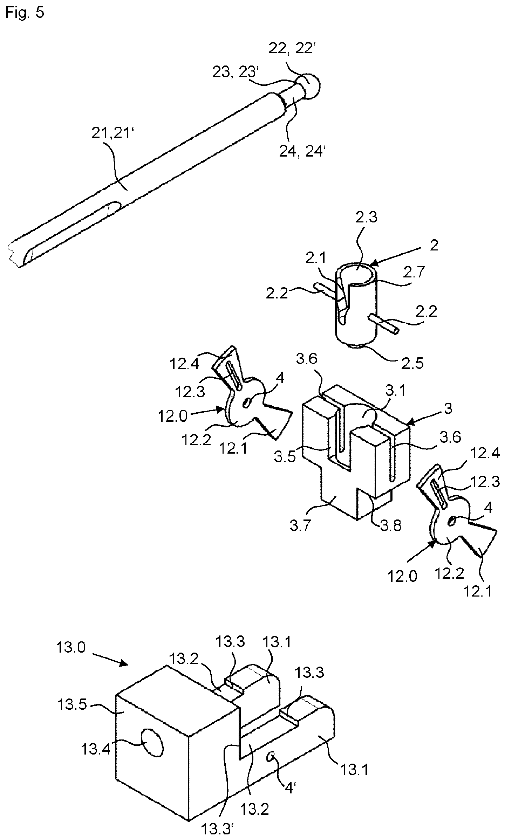

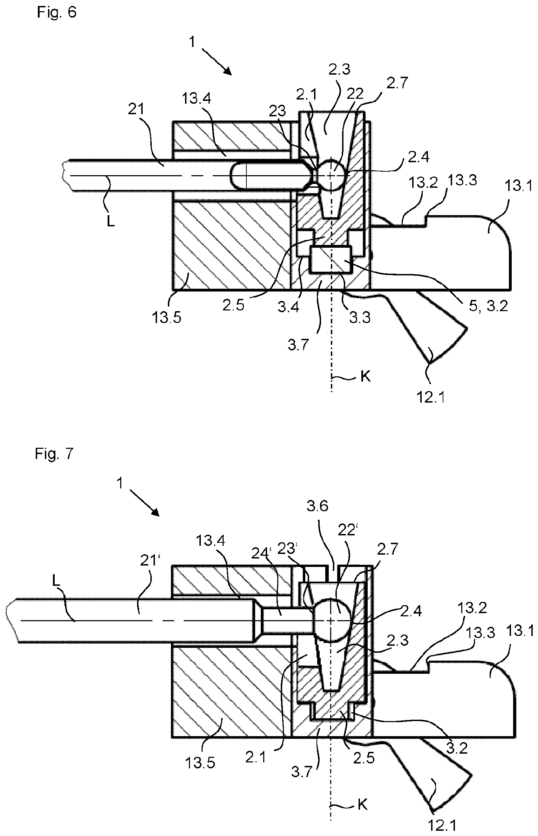

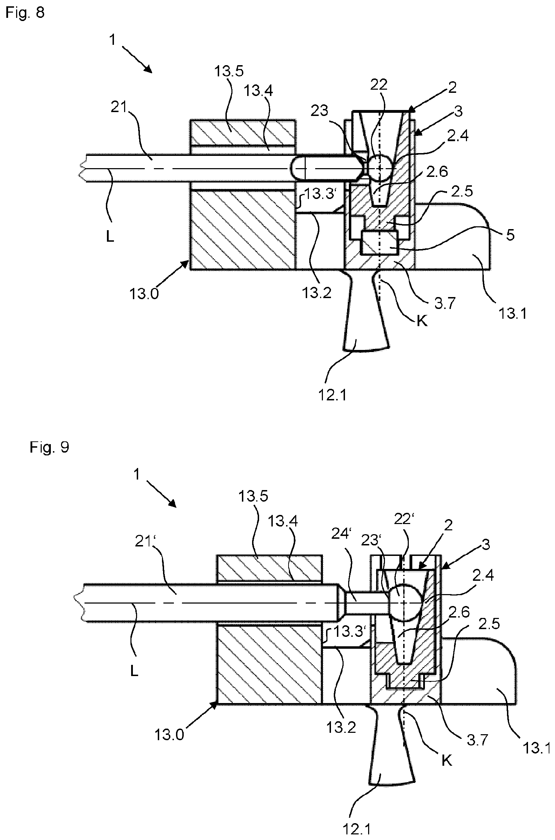

Das Kraftübertragungselement 21, 21', das jeweils mit der Kupplungsvorrichtung 1 in Eingriff treten kann und von dem in Fig. 5 ein Abschnitt mit dem Verbindungselement 22, 22' am proximalen Ende zu sehen ist, kann wahlweise ein erstes Kraftübertragungselement 21 (Fig. 6, 8) oder ein zweites Kraftübertragungselement 21' (Fig. 7, 9) (oder ein weiteres, nicht dargestelltes Kraftübertragungselement) sein, die sich bezüglich der durch sie übertragbare Kraft unterscheiden. Die vorliegenden Kraftübertragungselemente 21, 21' sind als Zug- und Schubstangen ausgebildet, wobei die Kraftübertragungselemente 21, 21' über ihre Länge im Wesentlichen mit einheitlichem, beispielsweise kreisrundem Querschnittprofil mit konstantem Durchmesser ausgebildet sein können. Ein Kraftübertragungselemente 21, 21' kann aber auch z. B. abgeflachte Abschnitte, wie in Fig. 5 gezeigt, oder Abschnitte mit anders abweichendem Querschnitt aufweisen, wenn diese für das Zusammenwirken mit einer weiter distalseitig liegende Instrumentenbauteilen vorteilhaft oder wünschenswert sind.

Das erste, zweite und optional jedes weitere Kraftübertragungselement 21, 21' unterscheidet sich dabei zumindest hinsichtlich seiner Stärke bzw. seines Durchmessers sowie einer Querschnittabmessung des jeweiligen Verbindungselements 22, 22', wie z. B. aus dem Vergleich von Fig. 6 und 7 ersichtlich ist, die gleiche Kupplungsvorrichtung 1 in Eingriff mit einem ersten Kraftübertragungselement 21 und einem zweiten Kraftübertragungselement 21' zeigen. Ferner kann die Kraft, die durch das jeweilige Kraftübertragungselement 21, 21' übertragbar ist, beispielsweise durch Materialwahl beeinflusst werden.

Welches Kraftübertragungselement 21, 21' in einem chirurgischen Instrument 100 aus Fig. 1 zum Einsatz kommt, hängt von dem eingesetzten Werkzeug 30 ab: Denn die in einem modularen Instrumentensystem zur Auswahl stehenden Werkzeuge unterscheiden sich - je nach Material und Anwendungszweck - hinsichtlich ihrer Belastungsgrenze. Die maximal tolerierbare Grenzkraft, die bei jedem Werkzeug vorbestimmt ist, sollte nicht überschritten werden, um die Funktionsfähigkeit des Werkzeugs zu erhalten und Beschädigung oder Bruch des Werkzeugs zu vermeiden. So ist die vorbestimmte Grenzkraft für Werkzeuge zum Schneiden bzw. Stanzen von Knochengewebe beispielsweise deutlich höher als die vorbestimmte Grenzkraft für Werkzeuge zum Präparieren von weicherem Gewebe. Dementsprechend wird ein Kraftübertragungselement, das zur Verbindung mit einem Werkzeug zum Schneiden bzw. Stanzen von Knochengewebe vorgesehen ist, zur Übertragung einer höheren Kraft ausgebildet sein und dazu eine größere Stärke bzw. einen größeren Durchmesser (wie das zweite Kraftübertragungselement 21') aufweisen als ein Kraftübertragungselement, das zur Verbindung mit einem Werkzeug zum Präparieren von weicherem Gewebe vorgesehen ist, und daher zur Übertragung einer geringeren Kraft ausgebildet ist und entsprechend eine geringere Stärke bzw. einen kleineren Durchmesser (als das erste Kraftübertragungselement 21) aufweist.

Daher können Werkzeug 30 und jeweiliges Kraftübertragungselement 21, 21' als passend zusammengestellte Einheiten in einem modularen Instrumentensystem bereitgestellt sein, müssen aber nicht. Ein modulares Instrumentensystem kann auch separat Werkzeuge und Kraftübertragungselemente aufweisen, die passend zusammengestellt werden können.

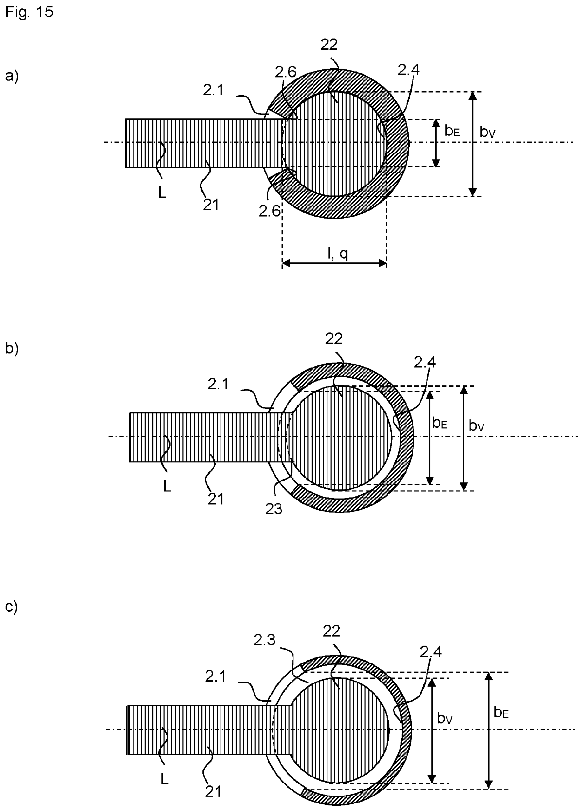

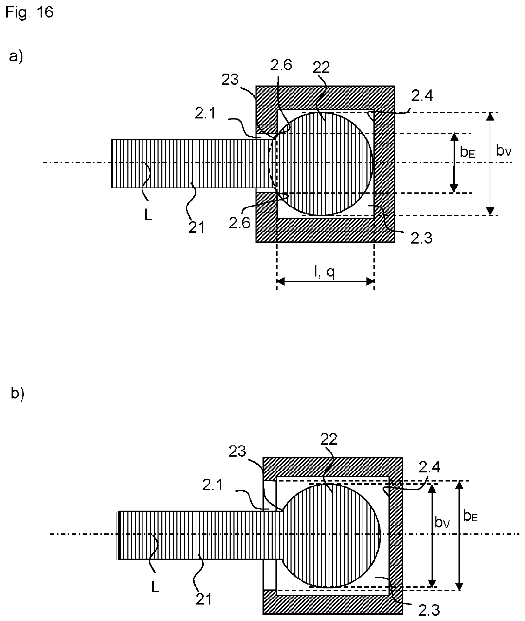

Zum Eingriff mit der erfindungsgemäßen Kupplungsvorrichtung 1 weist jedes Kraftübertragungselement 21, 21' an seinem proximalen Ende ein Verbindungselement 22, 22', das durch einen Absatz 23, 23' von dem stangenförmigen Kraftübertragungselement 21, 21' abgegrenzt ist. In bevorzugten Ausführungsformen ist das Verbindungselement 22, 22' kugelförmig, wie Figuren 5 bis 9 zeigen. Der Absatz 23, 23' zum kugelförmigen Verbindungselement 22, 22' wird dabei durch einen Halsabschnitt 24, 24' des Kraftübertragungselements 21, 21' mit verringerter Querschnittsabmessungen bereitgestellt. Der Halsabschnitt 24, 24' weist einen Durchmesser auf, der kleiner ist als der Durchmesser des Verbindungselements 22, 22' und des restlichen stangenförmigen Kraftübertragungselements 21, 21'. In den gezeigten Ausführungsformen hat das Kraftübertragungselements 21, 21' abgesehen von dem Halsabschnitt 23, 23' denselben Durchmesser wie das Verbindungselement 22, 22'. Abweichend dazu kann allerdings ein Absatz 23 auch ohne Halsabschnitt gebildet werden, wenn der Durchmesser des Verbindungselements 22 größer ist als der Durchmesser des Kraftübertragungselements 21 siehe Figuren 15 und 16.

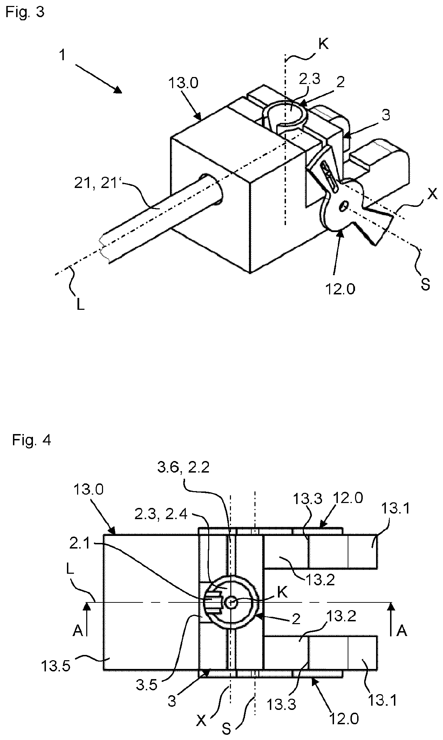

Die erfindungsgemäße Kupplungsvorrichtung 1, wie in Fig. 3 bis 14 gezeigt, ist zur Kopplung eines solchen Kraftübertragungselements 21, 21', das eine Längsachse L definiert, mit einem Griffteil 12 des chirurgischen Instruments 100 vorgesehen, das um eine zur Längsachse L rechtwinklige Schwenkachse S beweglich ist. Dazu weist die Kupplungsvorrichtung 1 ein Kupplungselement 2 und ein damit zusammenwirkendes Schieberelement 3 auf. Das Kupplungselement 2 hat eine Ausnehmung 2.3 zur Aufnahme des abgesetzten Verbindungselements 22, 22', und das Schieberelement 3 ist zum Zusammenwirken mit dem beweglichen Griffteil 12 und zur Führung in Richtung der Längsachse L ausgebildet.

Das Schieberelement 3 weist eine Führungsaufnahme 3.1 zur Aufnahme des Kupplungselements 2 auf, die entlang einer Kopplungsachse K ausgebildet ist, die rechtwinklig zu der Längsachse L und rechtwinklig zu der Schwenkachse S verläuft. So kann das Kupplungselement 2 in der nach oben offenen Führungsaufnahme 3.1 des Schieberelements 3 entlang der Kopplungsachse K bewegt werden. Zur Führung dieser Bewegung korrespondiert die Innenkontur der Führungsaufnahme 3.1 zumindest teilweise mit der Außenkontur des Kupplungselements 2. In dem Kupplungselement 2 verläuft die Ausnehmung 2.3 zur Aufnahme des Verbindungselements 22, 22', das am proximalen Ende des Kraftübertragungselements 21, 21' vorliegt, ebenfalls entlang der Kopplungsachse K.

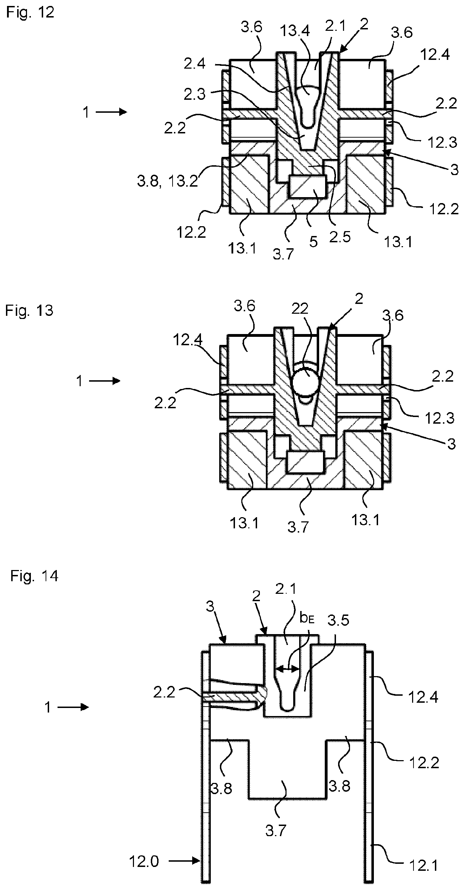

Dabei verjüngt sich der Querschnitt der Ausnehmung 2.3 ausgehend von der nach oben weisenden Öffnungsseite 2.7 entlang der Kopplungsachse K. Zudem weist das Kupplungselement 2 distalseitig einen Einschnitt 2.1 auf, der mit der Ausnehmung 2.3 verbunden ist und sich ebenfalls in einer Richtung parallel zu der Kopplungsachse K verjüngt. Der Einschnitt 2.1 weist damit an der Öffnungsseite 2.7 seine größte Breite b_{E} auf (vgl. Fig. 14). Die die Breite b_{E} des Einschnitts 2.1 parallel zur Kopplungsachse K kann sich beispielsweise konstant verringern oder, wie z. B. in Fig. 14 zu sehen, nur abschnittsweise in einem mittleren Bereich des Einschnitts. Abweichend dazu kann die Verjüngung des Einschnitts auch nichtkonstant und/oder in mehreren Abschnitten erfolgen.

Da das Kupplungselement 2 in der Führungsaufnahme 3.1 des Schieberelements 3 aufgenommen ist, ist die Führungsaufnahme 3.1 des Schieberelements 3 distalseitig mit einer Durchtrittsöffnung 3.5 verbunden, die den Einschnitt 2.1 des Kopplungselements 2 um Durchtritt eines jeweiligen Kraftübertragungselements 21, 21' mit Verbindungselement 22, 22' überlagert.

Die Abmessungen des sich verjüngenden Querschnitts der Ausnehmung 2.3 und des sich verjüngenden Einschnitts 2.1 des Kupplungselements 2 sind so dimensioniert, dass unterschiedlich große Verbindungselemente 22, 22' unterschiedlicher Kraftübertragungselemente 21, 21' in die Ausnehmung 2.3 aufgenommen werden können. Dazu ist das Kupplungselement 2 in der Kupplungsvorrichtung 1 senkrecht zu der Längsachse L des Kraftübertragungselements 21, 21' entlang der Kopplungsachse K in der Führungsaufnahme 3.1 des Schieberelements 3 beweglich. Auf diese Weise variieren die Querschnittabmessungen der Ausnehmung 2.3 und die Breite des Einschnitts 2.1 auf Höhe der Längsachse L in Abhängigkeit der Lage des Kupplungselements 2 entlang der Kopplungsachse K in der Führungsaufnahme 3.1.

Für ein gegebenes Kraftübertragungselement 21, 21' mit einem Verbindungselement 22, 22', das definierte Abmessungen aufweist, kann das Kupplungselement 2 entlang der Kopplungsachse K in der Führungsaufnahme 3.1 zwischen einer Eingriffsposition, in der die Verbindung zwischen dem Kraftübertragungselement 21, 21' und dem Griffteil hergestellt ist, und einer Freigabeposition bewegt werden, in der das Kraftübertragungselement 21, 21' nicht mit dem Griffteil verbunden ist.

In der Eingriffsposition des Kupplungselements 2 korrespondiert, wie insbesondere in Fig. 15a und 16a zu sehen ist, auf Höhe der Längsachse L die jeweilige Querschnittsabmessung q der Ausnehmung 2.3 in Richtung der Längsachse L mit der jeweiligen Längsschnittabmessung 1 des Verbindungselements 22, das somit an der dem Einschnitt 2.1 gegenüberliegenden Innenwand 2.4 zur Anlage kommt. Ferner ist auf Höhe der Längsachse L die Breite b_{E} des Einschnitts 2.1 kleiner ist als die Breite bv des Verbindungselements 22, sodass das Verbindungselement 22 den Einschnitt 2.1 hintergreift und in der Ausnehmung 2.3 an den Innenwandabschnitten 2.6 zur Anlage kommt, die den Einschnitt 2.1 begrenzen. Auf diese Weise steht das Verbindungselement 22 in Längsrichtung spielfrei mit dem Kupplungselement 2 in Eingriff. Entsprechendes gilt auch für die in Fig. 6 bis 9, 12 dargestellten Kupplungsvorrichtungen 1, die mit unterschiedlichen Verbindungselementen 22, 22' unterschiedlicher Kraftübertragungselemente 21, 21' in Eingriff stehen.

Da es sich in Fig. 15 um ein kugelförmiges Verbindungselement 22 und eine Ausnehmung 2.3 mit kreisförmigem Querschnitt handelt, entsprechen dabei definitionsgemäß die Längsschnittabmessung 1 des Verbindungselements 22 und die Querschnittabmessung q der Ausnehmung 2.3 auf Höhe und in Richtung der Längsachse L in der Eingriffsposition in Fig. 15a dem Durchmesser der Ausnehmung 2.3 und des Verbindungselements 22 auf Höhe der Längsachse L.

Fig. 16 hingegen zeigt ein kugelförmiges Verbindungselement 22, das in einer Ausnehmung 2.3 mit polygonalem, hier rechteckigen Querschnitt aufgenommen ist. Die Ausnehmung 2.3 mit solch polygonaler Querschnittsform kann beispielsweise als Pyramidenstumpf ausgeformt sein, bei dem alle Seitenwände aufeinander zulaufen. Alternativ ist es auch möglich, dass nur die proximale, dem Einschnitt gegenüberliegende Innenwand 2.4 und die den Einschnitt begrenzenden Innenwandabschnitte 2.6 aufeinander zulaufen, sodass die Ausnehmung ein trapezförmiges Verjüngungsprofil aufweist. Dort entspricht die Querschnittabmessung q der Ausnehmung 2.3 dem Abstand in Längsrichtung L zwischen der proximalen Innenwand 2.4, die dem Einschnitt 2.1 gegenüberliegt, und den distalen Innenwandabschnitten 2.6, die den Einschnitt 2.1 begrenzen. Dieser Abstand ist auf Höhe der Längsachse L für ein vorgegebenes kugelförmiges Verbindungselement 22 so dimensioniert, dass das kugelförmige Verbindungselement 22 proximalseitig an der proximalen Innenwand 2.4 und distalseitig an den distalen Innenwandabschnitten 2.6 zur Anlage kommt. Im dargestellten Beispiel kontaktiert das Verbindungselement 22 die distalen Innenwandabschnitte 2.6 proximalseitig des Absatz 23 der Stelle des Verbindungselements 22, deren Breite der Breite b_{E} des Einschnitts 2.1 entspricht, die größer als die Breite des Absatzes 23 ist.

Fig. 15c und 16b zeigen eine jeweils entsprechende Freigabeposition des Kupplungselements 2, in der auf Höhe der Längsachse L die Breite b_{E} des Einschnitts 2.1 größer ist als die Breite bv des Verbindungselements 22, sodass das Verbindungselement 22 durch den Einschnitt 2.1 in Richtung der Längsachse L herausgezogen werden kann. Ferner ist durch Vergleich mit Fig. 15a und 16a zu sehen, dass die Querschnittsabmessungen der Ausnehmung 2.3 in der Freigabeposition des Kupplungselements 2 größer sind als in der Eingriffsposition. Zur Überführung von der Eingriffsposition in Fig. 15a und 16a in die Freigabeposition nach Fig. 15c und 16b wird das Kupplungselement 2 senkrecht zur Zeichenebene bewegt.

In Fig. 15b ist zudem eine zwischen Eingriffs- und Freigabeposition aus Fig. 15a, c liegende Nichteingriffs- bzw. Übergangsposition des Kupplungselements 2 verdeutlicht, in der zwar die Querschnittsabmessungen der Ausnehmung 2.3 auf Höhe der Längsachse L größer sind als die Längsschnittabmessungen des Verbindungselements 22. Da jedoch die Breite b_{E} des Einschnitts 2.1 auf Höhe der Längsachse L dort kleiner ist als die Breite bv des Verbindungselements 22, kann das Verbindungselement 22 noch nicht durch den Einschnitt 2.1 herausgezogen werden, obwohl das Verbindungselement 22 nicht mehr in Längsrichtung spielfrei in der Ausnehmung 2.3 anliegt. In einer Abwandlung der Kupplungsvorrichtung 1, in der das Kupplungselement in der Freigabeposition entlang der Kupplungsachse K so weit von der Längsachse L wegbewegt vorliegt, dass das Verbindungselement sich oberhalb der Öffnungsseite, d. h. nicht mehr in der Ausnehmung befindet, kann die Einschnittbreite an der Öffnungsseite des Kupplungselements kleiner sein als die Breite des Verbindungselements.

Besonders vorteilhaft sind die in Fig. 3 bis 15 gezeigten Ausführungsformen der Kupplungsvorrichtung 1, bei denen die Ausnehmung 2.3 im Kupplungselement 2 kegelstumpfförmig mit einem kreisförmigen Querschnitt zum Eingriff mit einem kugelförmigen Verbindungselement 22, 22' ausgebildet ist. Die Querschnittsabmessung q der Ausnehmung 2.3 in Richtung der Längsachse L entspricht dann dem Durchmesser des jeweiligen kreisförmigen Querschnitts. Und die Längsschnittabmessung l des Verbindungselements 22, 22' entspricht dem Durchmesser des kugelförmigen Verbindungselements 22, 22'. Hierdurch wird eine spielfreie Aufnahme des Verbindungselements 22, 22' in der Ausnehmung 2.3 des Kupplungselements 2 ermöglicht, ohne dass ein Verklemmen des Verbindungselements 22, 22' zu befürchten ist, da das Verbindungselement 22, 22' nicht flächig, sondern nur an einer Umfangslinie in der Ausnehmung zur Anlage kommt.

In der in Fig. 3 bis 14 gezeigten Ausführungsform ist zur Führung des Schieberelements 3 in Längsrichtung eine Lagerkomponente 13.0 der Kupplungsvorrichtung 1 mit zwei Führungsschienen 13.1 ausgebildet, die sich parallel zu der Längsachse L erstrecken. Die beiden Führungsschienen 13.1 erstrecken sich von einem Blockabschnitt 13.5 der Lagerkomponente 13.0, der entlang der Längsachse L eine Durchgangsöffnung 13.4 aufweist, durch die sich das jeweilige Kraftübertragungselement 22, 22' längsbeweglich erstreckt. Die Lagerkomponente 13.0 der Kupplungsvorrichtung 1 ist entsprechend zur Anordnung in dem Gehäuse 13 der Griffvorrichtung 10 des chirurgischen Instruments 100 vorgesehen.

Das Schieberelement 3 weist auf einer von der Führungsaufnahme 3.1 abgewandten Seite einen Führungszapfen 3.7 auf, der entlang der Kopplungsachse K zwischen zwei Auflagerabschnitten 3.8 als Führungsprofil ausgebildet ist. Die Breite des quaderförmigen Führungszapfens 3.7 entspricht dem Abstand zwischen den Führungsschienen 13.1, sodass der Führungszapfen 3.7 zwischen den Führungsschienen 13.1 aufgenommen werden kann. Die beidseitig des Zapfens 3.7 abgesetzten Auflagerabschnitte 3.8 sind korrespondierend zu den Führungsschienen 13.1 ausgebildet und liegen gleitend auf den Führungsschienen auf 3.1. Dabei weist jede Führungsschiene 13.1 einen Führungsabschnitt 13.2 auf, der für das Schieberelement 3 einen Schiebeweg definiert, dessen Länge durch einen proximalen Anschlag 13.3 und einen distalen Anschlag 13.3' begrenzt ist. Im gezeigten Beispiel wird der distale Anschlag 13.3' durch den Blockabschnitt 13.5 gebildet.

Zur Verbindung mit dem beweglichen Griffteil 12 weist die Kupplungsvorrichtung 1 in den in Fig. 3 bis 14 gezeigten Ausführungsformen zwei beidseitig an der Lagerkomponente 13.0 um die Schwenkachse S schwenkbar gelagerte Schwenkbügel 12.0 auf. Jeder Schwenkbügel 12.0 weist hierbei einen Verbindungsabschnitt 12.1 zur Verbindung mit dem beweglichen Griffteil 12 und einen Mitnehmerabschnitt 12.4 auf, um die Bewegung des Griffteils 12 auf das Schieberelement 3 zu übertragen. Zwischen dem Verbindungsabschnitt 12.1 und dem Mitnehmerabschnitt 12.4 befindet sich ein Zentralabschnitt 12.2, der als Schwenklagervorrichtung eine Schwenklageröffnung 4 aufweist, durch die die Schwenkachse S verläuft. Der Verbindungsabschnitt 12.1 und der Mitnehmerabschnitt 12.4 verlaufen in radialer Richtung zur Schwenklageröffnung 4 und sind im gezeigten Beispiel unter einem Winkel von etwa 160° zueinander angeordnet. Abweichend dazu sind aber auch eine diametrale Anordnung von Verbindungsabschnitt und Mitnehmerabschnitt am Zentralabschnitt oder eine gewinkelte Anordnung mit einem von 160° abweichenden Winkel möglich; dies ist u. a. von der Länge des Schiebewegs sowie der Gestaltung und Anordnung des Griffteils 12 abhängig.

In einem Bereich unterhalb des Führungsabschnitts 13.2 weist jede Führungsschiene 13.1 als Lagervorrichtung eine Lageröffnung 4', zu sehen in Fig. 5, die koaxial zur Schwenkachse S zur Zusammenwirkung mit der Schwenklagervorrichtung 4 ausgebildet ist, damit der Schwenkbügel 12.0 um die Schwenkachse S schwenkbar an der Lagerkomponente 13.0 angelenkt werden kann. Nicht dargestellt in den Figuren ist ein mit der Schwenklageröffnung 4 und der Lageröffnung 4' zur Bildung des Schwenkgelenks zusammenwirkendes Achselement. Dazu kann ein Achselement beispielsweise mit einem Achsenende entweder in der Schwenklageröffnung 4 oder der Lageröffnung 4' drehfest angeordnet sein und mit dem anderen Achsenende drehbar in der jeweils anderen Öffnung, d. h. der Lageröffnung 4' oder der Schwenklageröffnung 4, gelagert sein. Oder das Achselement kann als freilaufende Achse konzipiert sein, und in beiden Öffnungen, Lageröffnung 4' und Schwenklageröffnung 4, drehbar gelagert sein. Abweichend zu dem dargestellten Beispiel kann in einer Variante eine von Lager- und Schwenklagervorrichtung anstelle einer Öffnung als Achsstummel ausgebildet sein, der in der anderen, als Öffnung ausgebildeten Lager- oder Schwenklagervorrichtung drehbar gelagert wird.

Das Zusammenwirken der Schwenkbügels 12.0 mit dem Schieberelement 3 wird über das in dem Schieberelement 3 aufgenommene Kupplungselement 2 bereitgestellt. Dazu weist das Kupplungselement 2 zwei diametral nach außen ragende Führungsstifte 2.2 auf, die eine zur Schwenkachse S parallele Achse X definieren (siehe Fig. 3, 4, 10). Für diese beiden Führungsstifte 2.2 weist das Schieberelement 3 zwei diametral mit der Führungsaufnahme 3.1 verbundene Führungsspalte 3.6 auf, die in einer durch die Achse X und die Kopplungsachse K definierten Ebene liegen. So werden beide Führungsstifte 2.2 in den Führungsspalten 3.6 in einer Richtung parallel zur Kopplungsachse K geführt, wenn das Kupplungselement 2 in der Führungsaufnahme 3.1 entlang der Kopplungsachse K bewegt wird.

Die Führungsstifte 2.2 sind dabei so dimensioniert, dass sie mit einem Endabschnitt an ihrem freien Ende aus dem Führungsspalt 3.6 des Schieberelements 3 herausragen, wie in Fig. 3 und insbesondere in Fig. 12 bis 14 zu sehen ist. Mit dem freien Endabschnitt ragen die Führungsstifte 2.2 in eine Führungsnut 12.3, die im Schwenkbügel 12.0 ausgebildet ist. Diese Führungsnut 12.3 erstreckt sich in dem Mitnehmerabschnitt 12.4 in radialer Richtung zu der Schwenklagervorrichtung 4, wie in Fig. 3, 5 und 10 zu sehen. Bei Betätigung am Griffteil 12 werden der Schwenkbügel 12.0 um die Schwenkachse S bewegt, wobei die Schwenkbewegung des Mitnehmerabschnitts 12.4 über den in der Führungsnut 12.3 aufgenommenen Führungsstift 2.2 in eine Längsbewegung des Schieberelements 3 über das darin aufgenommene Kupplungselement 2 umgesetzt wird.

Dies ist in Fig. 6 bis 9 für die mit zwei unterschiedlichen Kraftübertagungselementen 22, 22' in Eingriff stehende Kupplungsvorrichtung 1 dargestellt: Fig. 6 und 7 zeigen das Schieberelement 3 am distalen Anschlag 13.3' des Führungsabschnitts 13.2 der Führungsschiene 13.1, wobei der Verbindungsabschnitt 12.1 des Schwenkbügels 12.0 in proximale Richtung verschwenkt ist. Nach Betätigung des beweglichen Griffteils 12 wird der damit verbundene Verbindungsabschnitt 12.1 in Fig. 8 und 9 in distale Richtung verschwenkt. Dabei wird der Mitnehmerabschnitt 12.4, der in Fig. 6 bis 9 von dem Schieberelement 3 und dem Kupplungselement 2 verdeckt ist, entsprechend in proximale Richtung verschwenkt. Diese Bewegung des Mitnehmerabschnitts 12.4 führt über die Kopplung des Kupplungselements 2 sowohl mit dem Mitnehmerabschnitt 12.4 als auch mit dem Schieberelement 3 zu der Längsbewegung des Schieberelements 3 entlang den Führungsschienen 13.1.

Das jeweils mit dem Kopplungselement 2 in Eingriff stehende Kraftübertragungselement 21, 21' folgt der Bewegung in Längsrichtung, wodurch ein Werkzeug 30 am distalen Ende eines chirurgischen Instruments 100 (vgl. Fig. 1) betätigt wird, beispielsweise zum Schließen der Maulteile von Präparier-, Fass- und Exzisionszangen, Stanzen und Scheren. Je nach Größe und Einsatzbereich sind für diese Werkzeuge unterschiedliche Grenzkräfte vorgegeben, die nicht überschritten werden sollen, um ein Bauteilversagen oder Bruch zu vermeiden. Daher gibt es in chirurgischen Instrumentensystemen unterschiedliche Kraftübertragungselemente, die zur Kopplung mit den Werkzeugen zur Übertragung unterschiedlicher Grenzkräfte ausgebildet sind, um eine Überlast am distalen Werkzeug zu verhindern.

Dazu sind in Fig. 6 bis 9 zwei unterschiedliche Kraftübertragungselemente 21, 21' dargestellt, die zur Übertragung unterschiedlicher Grenzkräfte ausgebildet sind und in Eingriff mit dem Kupplungselement 2 der Kupplungsvorrichtung 1 stehen. Das in Fig. 6 und 8 dargestellte erste Kraftübertragungselement 21 ist zur Übertragung einer ersten Grenzkraft ausgebildet, die kleiner ist als die zweite Grenzkraft, die mit dem zweiten Kraftübertragungselement 21' übertragen werden kann, das in Fig. 7 und 9 zu sehen ist. Beide Kraftübertragungselemente 21, 21' weisen an ihrem proximalen Ende ein kugelförmiges Verbindungselement 22, 22' auf, das durch einen Halsabschnitt 23, 23' mit verringertem Durchmesser von dem stangeförmigen Kraftübertragungselement 21, 21' abgesetzt ist. Das erste Kraftübertragungselement 21, das zur Übertragung der kleineren Grenzkraft ausgebildet ist, weist einen kleineren Durchmesser als das zweite Kraftübertragungselement 21' auf, wobei das erste kugelförmige Verbindungselement 22 entsprechend einen kleineren Durchmesser aufweist als das zweite kugelförmige Verbindungselement 22. Und da die Eingriffsposition des Kupplungselements 2 entlang der Kopplungsachse K davon abhängig ist, dass der Querschnittsdurchmesser der Ausnehmung 2.3 auf Höhe der Längsachse L dem Durchmesser des jeweiligen Verbindungselements 22, 22' entspricht, unterscheidet sich auch die Eingriffsposition des Kupplungselements 2 mit dem ersten Verbindungselement 22 entlang der Kupplungsachse K von der Eingriffsposition des Kupplungselements 2 mit dem zweiten Verbindungselement 22.

Fig. 6 und 8 zeigen das Kupplungselement 2 in einer ersten Eingriffsposition mit dem ersten, kleineren Verbindungselement 22, während in Fig. 7 und 9 das Kupplungselement 2 in einer zweiten Eingriffsposition mit dem zweiten, größeren Verbindungselement 22' dargestellt ist. Da sich die Ausnehmung 2.3 kegelstumpfförmig verjüngt, wird das Kupplungselement 2 für die erste Eingriffsposition entlang der Kupplungsachse K weiter in Richtung der Längsachse L bewegt als für die zweite Eingriffsposition. Das erste, kleinere Verbindungselement 22 ist damit in der ersten Eingriffsposition, in der es an dem proximalen Innenwandabschnitt 2.4 zur Anlage kommt und den Einschnitt 2.1 an den distalen Innenwandabschnitten 2.6 hintergreift, weiter von der Öffnungsseite der Ausnehmung 2.3 entfernt als das zweite, größere Verbindungselement 22. Dieses befindet sich in der zweiten Eingriffsposition, in der es an dem proximalen Innenwandabschnitt 2.4 zur Anlage kommt und den Einschnitt 2.1 an den distalen Innenwandabschnitten 2.6 hintergreift, näher an der Öffnungsseite der Ausnehmung 2.3.

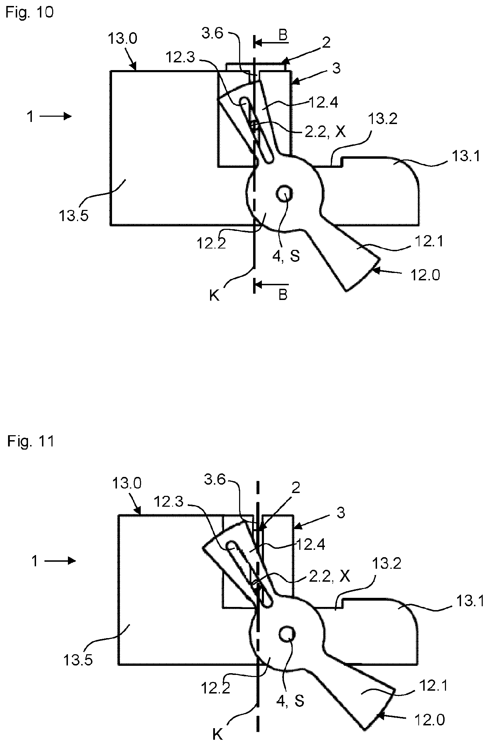

In Bezug auf das Schieberelement 3 ist das Kupplungselement 2 in der zweiten Eingriffsposition weiter in der Führungsaufnahme 3.1 aufgenommen als in der ersten Eingriffsposition. Hierdurch wird bedingt, dass der Abstand zwischen der Schwenkachse S und der Achse X, die durch die Führungsstifte 2.2 des Kupplungselements 2 definiert wird, in der ersten Eingriffsposition (Fig. 10) größer ist als in der zweiten Eingriffsposition (Fig. 11). Damit variiert die Position der Führungsstift-Achse X in Bezug auf die parallel verlaufende Schwenkachse S in Abhängigkeit der Abmessungen des Verbindungselements 22, 22' des jeweils eingesetzten Kraftübertragungselements 21, 21'. Die Position der Schwenkachse S an der Lagerkomponente 13.0 und die Abmessungen des Verbindungsabschnitts 12.1 des Schwenkbügels 12.0 und des damit verbundenen Griffteils 12 hingegen sind festgelegt.

In Bezug auf die Schwenkachse S besteht ein Hebelverhältnis zwischen einem ersten, konstanten Hebel, der zwischen der Schwenkachse S und dem Griffteil 12 definiert wird, an dem die Betätigungskraft wirkt, und einem zweiten, variablen Hebel, der durch den Abstand der Schwenkachse S von der Führungsstift-Achse X bei in Eingriffsposition befindlichem Kupplungselement 2 definiert ist. Mit der Eingriffsposition, die in Abhängigkeit der Abmessungen des Verbindungselements 22, 22' variiert, ändert sich dieses Hebelverhältnis, wodurch das Kräfteverhältnisses der an dem beweglichen Griffteil aufgebrachten Betätigungskraft zu einer axialen Bewegungskraft des Kraftübertragungselements 21, 21' in gewissem Umfang anpassbar ist.

Bei Eingriff des kleineren Verbindungselements 22 mit dem Kupplungselement 2 in der ersten Eingriffsposition (Fig. 10) ist der zweite Hebel größer als bei Eingriff des größeren Verbindungselements 22' mit dem Kupplungselement 2 in der zweiten Eingriffsposition (Fig. 11). Folglich ist das Hebelverhältnis zwischen dem konstanten ersten Hebel und dem zweiten Hebel in der ersten Eingriffsposition (Fig. 10) kleiner als in der zweiten Eingriffsposition (Fig. 11). Entsprechend wird bei gleicher Betätigungskraft am Griffteil 12 auf das kleinere Kraftübertragungselement 21 in der ersten Eingriffsposition eine kleinere Kraft zur Längsbewegung übertragen als auf das größere Kraftübertragungselement 21' in der zweiten Eingriffsposition. Dies sorgt für einen zusätzlichen Schutz der distalen Bauteile wie dem Werkzeug und sichert dessen Funktionsfähigkeit.

Und damit das Kupplungselement 2 in der jeweiligen Eingriffsposition gehalten wird, weist die Kupplungsvorrichtung 1 ein Federelement 5 auf, das z. B. in Fig. 6, 8 und 12 zu sehen ist.

Das Federelement 5 übt auf das Kupplungselement 2 eine in Richtung der Kopplungsachse K gerichtete Kraft aus, die Kupplungselement 2 in der Führungsaufnahme 3.1 nach oben drückt. Dabei stützt sich das Federelement 5 im Schieberelement 3 unten am Boden 3.3 in einer Aufnahmeausnehmung 3.2 ab, die sich unter Ausbildung eines ringförmigen Absatzes 3.4 an die Führungsaufnahme 3.1 anschließt. Das Kupplungselement 2 weist einen Stutzen 2.5 auf, an dem ein Federelement 5 wie eine Schraubenfeder gesichert werden kann. Der Stutzen 2.5 ist an der Unterseite des Kupplungselements 2 koaxial zur Kupplungsachse K unter Ausbildung einer Schulter abgesetzt und korrespondierend zu der Aufnahmeausnehmung 3.2 ausgebildet. Der Stutzen 2.5 kann somit je nach Lage der Eingriffsposition teilweise oder in die Aufnahmeausnehmung 3.2 aufgenommen werden, wobei das Federelement 5 komprimiert wird.

Daher kann auch die in Fig. 7 und 9 gezeigte Kupplungsvorrichtung 1 ein Federelement 5 aufweisen, das allerdings in der Darstellung aufgrund der Anordnung des Kupplungselements 2 in der zweiten Eingriffsposition nicht zu sehen ist. Denn dort ist der Stutzen 2.5 fast vollständig in der Aufnahmeausnehmung 3.2 aufgenommen, sodass das Federelement 5 maximal komprimiert vorliegt.

Es versteht sich, dass die erfindungsgemäße Kupplungsvorrichtung 1 auch zum Eingriff mit weiteren, nicht dargestellten Kraftübertragungselementen mit Verbindungselementen geeignet ist, deren Abmessungen von den Abmessungen der dargestellten Verbindungselementen abweichen, z. B. dazwischen liegen. Die Kupplungsvorrichtung 1 kann mit jedem Verbindungselement in Eingriff treten, dessen Durchmesser einer Querschnittabmessung an einer Stelle der Ausnehmung 2.3 entspricht und größer ist als die Breite des Einschnitts 2.1 an dieser Stelle. Wenn diese Stelle des Kupplungselements 2 auf Höhe der Längsachse L zu liegen kommt, hat das Kupplungselement 2 eine Eingriffsposition mit dem jeweiligen Verbindungselement erreicht. Damit kann eine Vielzahl von Bauteilkombinationen bezüglich Werkzeug und Kraftübertragungselement eines chirurgischen Instruments realisiert werden, die eine spielfreie Verbindung des Kraftübertragungselements mit dem Griffteil aufweisen. Zudem trägt dass mit der Eingriffsposition variable Hebelverhältnis dazu bei, das Kraftübertragungselement und das damit verbundene distale Werkzeug sowie ggf. weitere Bauteile vor Überlast bei Betätigung des Griffteils mit zu großer Kraft zu schützen. Ferner werden Zusammenbau und Demontage eines chirurgischen Instruments durch eine erfindungsgemäße Kupplungsvorrichtung vereinfacht.

Zum Zusammenbau eines chirurgischen Instruments 100 wie in Fig. 1 werden zunächst ein Werkzeug 30 und ein dazu passendes Kraftübertragungselement 21, 21' mit einem abgesetzten Verbindungselement 22, 22' am proximalen Ende sowie ein Instrumentenschaft 20 und eine Griffvorrichtung 10 ausgewählt, die ein um ein bewegliches Griffteil 12 und eine Kupplungsvorrichtung 1 aufweist, die der Ausführungsform aus Fig. 3 bis 14 entsprechen kann. Das Werkzeug 30 wird mit dem distalen Ende des Kraftübertragungselements 21, 21' verbunden, das zur Übertragung einer für ein Werkzeug 30 vorbestimmten maximalen Grenzkraft ausgebildet ist. Das Kraftübertragungselement 21, 21' wird durch den Instrumentenschaft 20 in die Griffvorrichtung 10 eingeführt, bis das Verbindungselement 22, 22' am proximalen Ende des Kraftübertragungselements 21, 21' die Kupplungsvorrichtung 1 erreicht. Davor oder danach kann eine Verbindung des Instrumentenschafts 20 distalseitig mit einem Werkzeughalter und proximalseitig mit der Griffvorrichtung 10 erfolgen.

Das Kupplungselement 2 ist in der Führungsaufnahme 3.1 des Schieberelements 3 entlang der Kopplungsachse K in einer Freigabeposition angeordnet oder wird in eine Freigabeposition bewegt, in der auf der Höhe der Längsachse L die Breite des Einschnitts 2.1 größer ist als die Breite des Verbindungselements 22, 22', wobei ggf. auch der Durchmesser bzw. die Querschnittabmessung der Ausnehmung 3.2 in Richtung der Längsachse L größer ist als der Durchmesser bzw. die Längsschnittabmessung des Verbindungselements 22, 22'. Dann wird das Verbindungselement 22, 22' am proximalen Ende des Kraftübertragungselements 21, 21' entlang der Längsachse L durch den Einschnitt 2.1 in die Ausnehmung 2.3 eingeführt, bis das Verbindungselement 22, 22' die Kupplungsachse K kreuzt. Dann kann das Kupplungselement 2 die entsprechende Eingriffsposition einnehmen, in der auf der Höhe der Längsachse L der Durchmesser bzw. die Querschnittsabmessung der Ausnehmung 2.3 in Richtung der Längsachse L mit dem Durchmesser bzw. der Längsschnittabmessung des Verbindungselements 22, 22' korrespondiert und die Breite des Einschnitts 2.1 kleiner ist als die Breite des Verbindungselements 22, 22'. Dann hintergreift das Verbindungselement 22, 22' den Einschnitt 2.1 und kommt in der Ausnehmung 2.3 in der Eingriffsposition zumindest an einer proximalen Innenwand 2.4 gegenüber dem Einschnitt 2.1 und an den distalen Innenwandabschnitten 2.6 zur Anlage, die den Einschnitt 2.1 begrenzen. Das chirurgische Instrument 100 ist dann einsatzbereit.

Die Demontage des chirurgischen Instruments 100 verläuft entsprechend in umgekehrter Reihenfolge. Dabei wird das Kupplungselement 2 aus der Eingriffsposition in eine Freigabeposition überführt, in der das Verbindungselement 22, 22' entlang der Längsachse L. durch den Einschnitt 2.1 aus der Ausnehmung 2.3 des Kupplungselements 2 und damit aus der Griffvorrichtung 10 herausgezogen werden kann. Weitere Demontageschritte können das Trennen des Instrumentenschafts 20 von der Griffvorrichtung 10 und/oder von einer distalen Werkzeughalterung und das Entfernen des Kraftübertragungselements 21, 21' aus dem Instrumentenschaft 20. Gegebenenfalls kann auch das Werkzeug 30 vom distalen Ende des Kraftübertragungselements 21, 21' getrennt werden, um die Bauteile des chirurgischen Instruments 100 getrennt zu entsorgen oder zur Wiederverwendung durch Reinigen und Desinfizieren aufzubereiten.

Die Zeichnungen, die Beschreibung und die Ansprüche enthalten zahlreiche Merkmale in Kombination. Es versteht sich, dass die vorstehend genannten Merkmale nicht nur in der jeweils angegebenen Kombination, sondern auch in anderen Kombinationen oder in Alleinstellung verwendbar sind, ohne den Rahmen der vorliegenden Erfindung zu verlassen. Die Erfindung bezieht sich auf eine Kupplungsvorrichtung 1 für ein chirurgisches Instrument 100, und eine Griffvorrichtung 10 hierfür, sowie auf entsprechende Montage- und Demontageverfahren. Das Instrument 100 weist ein Kraftübertragungselement 21, 21', das eine Längsachse L definiert und an einem proximalen Ende ein abgesetztes Verbindungselement 22, 22' aufweist, und eine Griffvorrichtung 10 mit einem Griffteil 12 auf, das um eine rechtwinklig zu der Längsachse L verlaufende Schwenkachse S beweglich ist. Dabei hat die Kupplungsvorrichtung 1 zur Verbindung des Kraftübertragungselements 21, 21' mit dem Griffteil 12 ein Kupplungselement 2 mit einer Ausnehmung 2.3 zur Aufnahme des Verbindungselements 22, 22' und ein mit dem Kupplungselement 2 zusammenwirkendes Schieberelement 3, das zum Zusammenwirken mit dem beweglichen Griffteil 12 und zur Bewegung in Richtung der Längsachse L ausgebildet ist. Das Kupplungselement 2 ist in einer Führungsaufnahme 3.1 des Schieberelements 3 entlang einer Kopplungsachse K bewegbar aufgenommen, und die Ausnehmung 2.3 in dem Kupplungselement 2 ist korrespondierend zu der Kopplungsachse K ausgebildet. Das Kupplungselement 2 hat einen Einschnitt 2.1, der mit der Ausnehmung 2.3 verbunden ist. Das Kupplungselement 2 ist entlang der Kopplungsachse K zwischen zumindest einer Freigabeposition und einer Eingriffsposition bewegbar.

### Bezugszeichenliste

- 1: Kupplungsvorrichtung
- 2: Kupplungselement
- 2.1: Einschnitt
- 2.2: Führungsstift
- 2.3: Ausnehmung
- 2.4: Proximaler Innenwandabschnitt
- 2.5: Stutzen
- 2.6: Distaler Innenwandabschnitt
- 2.7: Öffnungsseite
- 3: Schieberelement
- 3.1: Führungsaufnahme für Kupplungselement
- 3.2: Aufnahmeausnehmung für Federelement
- 3.3: Boden
- 3.4: Absatz
- 3.5: Durchtrittsöffnung für Kraftübertragungselement
- 3.6: Führungsspalt für Führungsstift
- 3.7: Führungszapfen
- 3.8: Auflagerabschnitt
- 4, 4': Schwenklagervorrichtung, Lagervorrichtung
- 5: Federelement
- 10: Griffvorrichtung
- 11: Unbewegliches Griffteil
- 12: Bewegliches Griffteil
- 12.0: Schwenkbügel
- 12.1: Verbindungsabschnitt
- 12.2: Zentralabschnitt
- 12.3: Führungsnut für Führungsstift
- 12.4: Mitnehmerabschnitt
- 13: Gehäuse
- 13.0: Lagerkomponente
- 13.1: Führungsschiene
- 13.2: Führungsabschnitt
- 13.3, 13.3': Anschlag
- 13.4: Durchgangsöffnung
- 13.5: Blockabschnitt
- 13.6: Aufnahmeraum
- 14: Verbindungsabschnitt
- 15: Proximaler Schaftendabschnitt
- 20: Schaft
- 21, 21': Kraftübertragungselement
- 22, 22': Verbindungselement
- 23, 23': Absatz
- 24, 24': Halsabschnitt
- 30: Werkzeug
- 100: Chirurgisches Instrument

- L: Längsachse
- K: Kopplungsachse
- S: Schwenkachse
- X: Achse Führungsstifte
- 1, q: Längsschnittabmessung, Querschnittsabmessung
- b_{E}, b_{V}: Breite des Einschnitts, Breite des Verbindungselements

## Patentansprüche

1. Kupplungsvorrichtung (1) für ein chirurgisches Instrument (100), das ein Kraftübertragungselement (21, 21'), das eine Längsachse (L) definiert und an einem proximalen Ende ein abgesetztes Verbindungselement (22, 22') aufweist, und eine Griffvorrichtung (10) mit einem Griffteil (12) aufweist, das um eine rechtwinklig zu der Längsachse (L) verlaufende Schwenkachse (S) beweglich ist, wobei die Kupplungsvorrichtung (1) zur Verbindung des Kraftübertragungselements (21, 21') mit dem beweglichen Griffteil (12) ein Kupplungselement (2) mit einer Ausnehmung (2.3) zur Aufnahme des Verbindungselements (22, 22') und ein mit dem Kupplungselement (2) zusammenwirkendes Schieberelement (3) aufweist, das zum Zusammenwirken mit dem beweglichen Griffteil (12) und zur Bewegung in Richtung der Längsachse (L) ausgebildet ist,
**dadurch gekennzeichnet, dass**
das Kupplungselement (2) in einer Führungsaufnahme (3.1) des Schieberelements (3) entlang einer Kopplungsachse (K) bewegbar aufgenommen ist, die rechtwinklig zu der Längsachse (L) verläuft,
die Ausnehmung (2.3) in dem Kupplungselement (2) korrespondierend zu der Kopplungsachse (K) ausgebildet ist und einen Querschnitt aufweist, der sich ausgehend in Richtung der Kopplungsachse (K) verjüngt, und
das Kupplungselement (2) einen Einschnitt (2.1) aufweist, der mit der Ausnehmung (2.3) verbunden ist, wobei sich eine Breite (b_{E}) des Einschnitts (2.1) in einer Richtung parallel zu der Kopplungsachse (K) verjüngt, und
wobei das Kupplungselement (2) entlang der Kopplungsachse (K) zwischen
- zumindest einer Freigabeposition, in der auf einer Höhe der Längsachse (L) die Breite (b_{E}) des Einschnitts (2.1) größer ist als eine Breite (bv) des Verbindungselements (22, 22'), und
- zumindest einer Eingriffsposition, in der auf der Höhe der Längsachse (L) die Querschnittsabmessung (q) der Ausnehmung (2.3) in Richtung der Längsachse (L) mit einer Längsschnittabmessung (l) des Verbindungselements (22, 22') korrespondiert und die Breite (b_{E}) des Einschnitts (2.1) kleiner ist als eine Breite (bv) des Verbindungselements (22, 22'), sodass das Verbindungselement (22, 22') den Einschnitt (2.1) hintergreift und in der Ausnehmung (2.3) zur Anlage kommt, bewegbar ist.

2. Kupplungsvorrichtung (1) nach Anspruch 1,
**dadurch gekennzeichnet, dass**
eine Querschnittsform der Ausnehmung (2.3) im Kupplungselement (2) senkrecht zur Kupplungsachse (K) mit einer Form einer Längsschnittfläche des Verbindungselements (22, 22') senkrecht zur Kupplungsachse (K) korrespondiert, wobei bevorzugt die Ausnehmung (2.3) im Kupplungselement (2) kegel- oder kegelstumpfförmig mit einem kreisförmigen Querschnitt und das Verbindungselement (22, 22') kugelförmig ausgebildet sind, wobei die Querschnittsabmessung (q) der Ausnehmung (2.3) in Richtung der Längsachse (L) ein Durchmesser des kreisförmigen Querschnitts ist und die Längsschnittabmessung (l) des Verbindungselements (22, 22') ein Durchmesser des kugelförmigen Verbindungselements (22, 22') ist.

3. Kupplungsvorrichtung (1) nach Anspruch 1 oder 2,
**dadurch gekennzeichnet, dass**
die Kupplungsvorrichtung (1) zumindest mit einem ersten Kraftübertragungselement (21) oder mit einem zweiten Kraftübertragungselement (21') in Eingriff bringbar ist, wobei das erste Kraftübertragungselement (21) ein abgesetztes erstes Verbindungselement (22) zur Übertragung einer ersten Grenzkraft und das zweite Kraftübertragungselement (21') ein abgesetztes zweites Verbindungselement (22') zur Übertragung einer zweiten Grenzkraft, die größer ist als die erste Grenzkraft, aufweisen, wobei die Längsschnittabmessung (l) des zweiten Verbindungselements (22') größer ist als die Längsschnittabmessung (l) des ersten Verbindungselements (22) und die Breite (bv) des zweiten Verbindungselements (22') größer ist als die Breite (bv) des ersten Verbindungselements (22), und
wobei das Kupplungselement (2) zum Eingriff
- mit dem ersten Kraftübertragungselement (21) entlang der Kopplungsachse (K) in einer ersten Eingriffsposition angeordnet wird, in der auf der Höhe der Längsachse (L) die Querschnittsabmessung (q) der Ausnehmung (2.3) in Richtung der Längsachse (L) mit der Längsschnittabmessung (l) des ersten Verbindungselements (22) korrespondiert und die Breite (b_{E}) des Einschnitts (2.1) kleiner ist als die Breite (bv) des ersten Verbindungselements (22), sodass das erste Verbindungselement (22) den Einschnitt (2.1) hintergreift und in der Ausnehmung (2.3) zur Anlage kommt, und
- mit dem zweiten Kraftübertragungselement (21') entlang der Kopplungsachse (K) in einer zweiten Eingriffsposition angeordnet wird, in der auf der Höhe der Längsachse (L) die Querschnittsabmessung (q) der Ausnehmung (2.3) in Richtung der Längsachse (L) mit der Längsschnittabmessung (l) des zweiten Verbindungselements (22') korrespondiert und die Breite (b_{E}) des Einschnitts (2.1) kleiner ist als die Breite (bv) des ersten Verbindungselements (22'), sodass das zweite Verbindungselement (22') den Einschnitt (2.1) hintergreift und in der Ausnehmung (2.3) zur Anlage kommt.

4. Kupplungsvorrichtung (1) nach zumindest einem der Ansprüche 1 bis 3,
**dadurch gekennzeichnet, dass**
die Kupplungsvorrichtung (1) eine Lagerkomponente (13.0) aufweist, die in einem Gehäuse (13) der Griffvorrichtung (10) vorliegt, wobei die Lagerkomponente (13.0) zwei Führungsschienen (13.1) aufweist, die sich parallel zu der Längsachse (L) erstrecken, und wobei das Schieberelement (3) auf einer von der Führungsaufnahme (3.1) abgewandten Seite ein Führungsprofil mit einem entlang der Kopplungsachse (K) ausgebildeten Führungszapfen (3.7) aufweist, der zwischen den zwei Führungsschienen (13.1) aufgenommen ist.

5. Kupplungsvorrichtung (1) nach Anspruch 4,
**dadurch gekennzeichnet, dass**
die Kupplungsvorrichtung (1) zumindest einen, bevorzugt zwei, an der Lagerkomponente (13.0) um die Schwenkachse (S) schwenkbar gelagerte(n) Schwenkbügel (12.0) zur Verbindung mit dem beweglichen Griffteil (12) aufweist, wobei der Schwenkbügel (12.0) zur Bereitstellung der Schwenkachse (S) eine Schwenklagervorrichtung (4) aufweist, und die Lagerkomponente (13.0) zumindest eine zu der Schwenkachse (S) koaxiale Lagervorrichtung (4') aufweist, die zur Zusammenwirkung mit der Schwenklagervorrichtung (4) zur schwenkbaren Lagerung des zumindest einen Schwenkbügels (12.0) an zumindest einer der Führungsschienen (13.1) ausgebildet ist.

6. Kupplungsvorrichtung (1) nach zumindest einem der Ansprüche 1 bis 5,
**dadurch gekennzeichnet, dass**
das Schieberelement (3) eine mit der Führungsaufnahme (3.1) verbundene Durchtrittsöffnung (3.5) aufweist, wobei die Durchtrittsöffnung (3.5) den Einschnitt (2.1) des Kupplungselements (2) überlagert, und wobei das Kupplungselement (2) auf einer Achse (X) parallel zur Schwenkachse (S) zumindest einen nach außen ragenden Führungsstift (2.2), bevorzugt zwei nach außen ragende Führungsstifte (2.2), aufweist, und das Schieberelement (3) zumindest einen Führungsspalt (3.6), bevorzugt zwei Führungsspalte (3.6), aufweist, der/die parallel zur Kopplungsachse (K) verläuft/verlaufen und mit der Führungsaufnahme (3.1) verbunden ist/sind, wobei der zumindest eine Führungsstift (2.2) in dem zumindest einen Führungsspalt (3.6) angeordnet ist.

7. Kupplungsvorrichtung (1) nach Anspruch 6,
**dadurch gekennzeichnet, dass**
der zumindest eine Führungsstift (2.2) zum Zusammenwirken mit dem zumindest einen Schwenkbügel (12.0) ausgebildet ist und mit einem Endabschnitt aus dem Führungsspalt (3.6) des Schieberelements (3) herausragt, wobei der Schwenkbügel (12.0) eine Führungsnut (12.3) aufweist, die in radialer Richtung zu der Schwenklagervorrichtung (4) verläuft und in der der Endabschnitt des Führungsstifts (2.2) aufgenommen ist.

8. Kupplungsvorrichtung (1) nach zumindest einem der Ansprüche 1 bis 7,
**dadurch gekennzeichnet, dass**
die Kupplungsvorrichtung (1) ein Federelement (5) aufweist, das eine Kraft in Richtung der Kopplungsachse (K) auf das Kupplungselement (2) ausübt und das Kupplungselement (2) in der Eingriffsposition hält.

9. Kupplungsvorrichtung (1) nach Anspruch 8,
**dadurch gekennzeichnet, dass**
das Federelement (5) in einer Aufnahmeausnehmung (3.2) des Schieberelements (3) angeordnet ist, die den Boden (3.3) aufweist und sich unter Ausbildung eines Absatzes (3.4) an die Führungsaufnahme (3.1) anschließt,
und/oder das Kupplungselement (2) einen von einer Öffnungsseite (2.7) der Ausnehmung (2.3) abgewandten Stutzen (2.5) zum Eingriff mit dem Federelement (5) aufweist.

10. Kupplungsvorrichtung (1) nach zumindest einem der Ansprüche 4 bis 9,
**dadurch gekennzeichnet, dass**
die Lagerkomponente (13.0) einen Blockabschnitt (13.5) aufweist, an dem die Führungsschienen (13.1) angeordnet sind, wobei der Blockabschnitt (13.5) eine Durchgangsöffnung (13.4) entlang der Längsachse (L) für das Kraftübertragungselement (22, 22') aufweist.

11. Griffvorrichtung (10) für ein chirurgisches Instrument (100) zur Anordnung an einem proximalen Ende eines Schafts (20), durch den sich ein Kraftübertragungselement (21, 21') erstreckt, das eine Längsachse (L) definiert und an einem proximalen Ende ein abgesetztes Verbindungselement (22, 22') aufweist, wobei die Griffvorrichtung (10) ein um eine Schwenkachse (S) bewegliches Griffteil (12) und eine Kupplungsvorrichtung (1) zur Verbindung des beweglichen Griffteils (12) mit dem Kraftübertragungselement (21, 21') aufweist,
**dadurch gekennzeichnet, dass**
die Kupplungsvorrichtung (1) eine Kupplungsvorrichtung (1) nach zumindest einem der Ansprüche 1 bis 10 ist.

12. Chirurgisches Instrument (100), das einen Schaft (20) aufweist, durch den sich ein entlang einer Längsachse (L) bewegbares Kraftübertragungselement (21, 21') mit einem abgesetzten Verbindungselement (22, 22') an einem proximalen Ende erstreckt, wobei das chirurgische Instrument (100) an einem proximalen Ende des Schafts (20) eine Griffvorrichtung (10) mit einem Griffteil (12), das um eine senkrecht zu der Längsachse (L) verlaufenden Schwenkachse (S) beweglich ist, und an einem distalen Ende des Schafts (20) ein Werkzeug (30) aufweist, das mit dem Kraftübertragungselement (21, 21') in Wirkverbindung steht, und wobei das chirurgische Instrument (100) eine Kupplungsvorrichtung (1) zur Verbindung des beweglichen Griffteils (12) mit dem Kraftübertragungselement (21, 21') aufweist,
**dadurch gekennzeichnet, dass**
die Kupplungsvorrichtung (1) eine Kupplungsvorrichtung (1) nach zumindest einem der Ansprüche 1 bis 10 ist.

13. Verfahren zum Zusammenbau eines chirurgisches Instruments (100) mit einer Griffvorrichtung (10) und einem Kraftübertragungselement (21, 21') mit einem abgesetzten Verbindungselement (22, 22') an einem proximalen Ende unter Verwendung einer Kupplungsvorrichtung (1) nach zumindest einem der Ansprüche 1 bis 10,
umfassend die Schritte
- Anordnen des Kupplungselements (2) in der Führungsaufnahme (3.1) des Schieberelements (3) entlang der Kopplungsachse (K) in der Freigabeposition, in der auf der Höhe der Längsachse (L) die Breite (b_{E}) des Einschnitts (2.1) größer ist als die Breite (bv) des Verbindungselements (22, 22');
- Einführen des Verbindungselements (22, 22') am proximalen Ende des Kraftübertragungselements (21, 21') entlang der Längsachse (L) in die Kupplungsvorrichtung (1) durch den Einschnitt (2.1) in die Ausnehmung (2.3);
- Überführen des Kupplungselements (2) in die Eingriffsposition, in der auf der Höhe der Längsachse (L) die Querschnittsabmessung (q) der Ausnehmung (2.3) in Richtung der Längsachse (L) mit der Längsschnittabmessung (l) des Verbindungselements (22, 22') korrespondiert und die Breite (b_{E}) des Einschnitts (2.1) kleiner ist als die Breite (bv) des Verbindungselements (22, 22'), sodass das Verbindungselement (22, 22') den Einschnitt (2.1) hintergreift und in der Ausnehmung (2.3) zur Anlage kommt.

14. Verfahren nach Anspruch 13, wobei das Kraftübertragungselement (21, 21'), das zur Übertragung einer vorbestimmten Grenzkraft ausgebildet ist, entsprechend einer für ein Werkzeug (30) vorbestimmten maximalen Grenzkraft ausgewählt wird, das an einem distalen Ende des Kraftübertragungselements (21, 21') angeordnet wird.

15. Verfahren zur Demontage eines chirurgisches Instruments (100) mit einer Griffvorrichtung (10), und einem Kraftübertragungselement (21, 21') mit einem abgesetzten Verbindungselement (22, 22') an einem proximalen Ende unter Verwendung einer Kupplungsvorrichtung (1) nach zumindest einem der Ansprüche 1 bis 10,
umfassend die Schritte
- Überführen des Kupplungselements (2) aus der Eingriffsposition, in der auf der Höhe der Längsachse (L) die Querschnittsabmessung (q) der Ausnehmung (2.3) in Richtung der Längsachse (L) mit der Längsschnittabmessung (l) des Verbindungselements (22, 22') korrespondiert und die Breite (b_{E}) des Einschnitts (2.1) kleiner ist als die Breite (bv) des Verbindungselements (22, 22'), in die Freigabeposition, in der auf der Höhe der Längsachse (L) die Breite (b_{E}) des Einschnitts (2.1) größer ist als die Breite (bv) des Verbindungselements (22, 22'); und
- Entfernen des Verbindungselements (22, 22') aus der Ausnehmung (2.3) des Kupplungselements (2) durch den Einschnitt (2.1) durch Herausziehen des Kraftübertragungselements (21, 21') entlang der Längsachse (L).

## Claims

1. A coupling device (l) for a surgical instrument (100), which has a force transmission element (21, 21'), which defines a longitudinal axis (L) and has a stepped connecting element (22, 22') at a proximal end, and a handle device (10) with a handle part (12), which is movable about a pivot axis (S) running at right angles to the longitudinal axis (L), wherein the coupling device (1) for connecting the force transmission element (21, 21') to the movable handle part (12) has a coupling element (2) with a recess (2.3) for receiving the connecting element (22, 22') and a sliding element (3) which cooperates with the coupling element (2) and is designed to cooperate with the movable handle part (12) and to move in the direction of the longitudinal axis (L),
**characterised in that** the coupling element (2) is received in a guide receptacle (3.1) of the sliding element (3) so as to be movable along a coupling axis (K) which runs at right angles to the longitudinal axis (L), the recess (2.3) in the coupling element (2) is designed to correspond to the coupling axis (K) and has a cross-section which tapers starting in the direction of the coupling axis (K), and the coupling element (2) has a notch (2.1) which is connected to the recess (2.3), wherein a width (b_{E}) of the notch (2.1) tapers in a direction parallel to the coupling axis (K), and wherein the coupling element (2) is movable along the coupling axis (K) between
- at least one release position in which, at a height of the longitudinal axis (L), the width (b_{E}) of the notch (2.1) is greater than a width (bv) of the connecting element (22, 22'), and
- at least one engagement position in which, at the height of the longitudinal axis (L), the cross-sectional dimension (q) of the recess (2.3) in the direction of the longitudinal axis (L) corresponds to a longitudinal sectional dimension (1) of the connecting element (22, 22') and the width (b_{E}) of the notch (2.1) is smaller than a width (bv) of the connecting element (22, 22') such that the connecting element (22, 22') engages behind the notch (2.1) and comes to rest in the recess (2.3).

2. The coupling device (1) according to claim 1,
**characterised in that**
a cross-sectional shape of the recess (2.3) in the coupling element (2) perpendicular to the coupling axis (K) corresponds to a shape of a longitudinal sectional surface of the connecting element (22, 22') perpendicular to the coupling axis (K), wherein preferably the recess (2.3) in the coupling element (2) is conical or frustoconical with a circular cross-section and the connecting element (22, 22') is spherical, wherein the cross-sectional dimension (q) of the recess (2.3) in the direction of the longitudinal axis (L) is a diameter of the circular cross-section and the longitudinal sectional dimension (1) of the connecting
element (22, 22') is a diameter of the spherical connecting element (22, 22').

3. The coupling device (1) according to claim 1 or 2,
**characterised in that**
The coupling device (1) can be brought into engagement with at least one first force transmission element (21) or with one second force transmission element (21'), wherein the first force transmission element (21) has a stepped first connecting element (22) for transmitting a first limit force and the second force transmission element (21') has a stepped second connecting element (22') for transmitting a second limit force which is greater than the first limit force, wherein the longitudinal sectional dimension (1) of the second connecting element (22') is greater than the longitudinal sectional dimension (1) of the first connecting element (22) and the width (bv) of the second connecting element (22') is greater than the width (bv) of the first connecting element (22), and wherein the coupling element (2) is arranged to engage
- with the first force transmission element (21) along the coupling axis (K) in a first engagement position in which, at the height of the longitudinal axis (L), the cross-sectional dimension (q) of the recess (2.3) in the direction of the longitudinal axis (L) corresponds to the longitudinal sectional dimension (1) of the first connecting element (22) and the width (b_{E}) of the notch (2.1) is smaller than the width (bv) of the first connecting element (22) such that the first connecting element (22) engages behind the notch (2.1) and comes to rest in the recess (2.3), and - with the second force transmission element (21') along the coupling axis (K) in a second engagement position in which, at the height of the longitudinal axis (L), the cross-sectional dimension (q) of the recess (2.3) in the direction of the longitudinal axis (L) corresponds to the longitudinal section dimension (1) of the second connecting element (22') and the width (b_{E}) of the notch (2.1) is smaller than the width (bv) of the first connecting element (22') such that the second connecting element (22') engages behind the notch (2.1) and comes to rest in the recess (2.3).

4. The coupling device (1) according to at least one of claims 1 to 3,
**characterised in that** the coupling device (1) has a bearing component (13.0) which is present in a housing (13) of the handle device (10), wherein the bearing component (13.0) has two guide rails (13.1) which extend parallel to the longitudinal axis (L), and wherein the sliding element (3) has, on a side facing away from the guide receptacle (3.1), a guide profile with a guide pin (3.7) which is formed along the coupling axis (K) and which is received between the two guide rails (13.1).

5. The coupling device (1) according to claim 4,
**characterised in that** the coupling device (1) has at least one, preferably two, pivot bracket(s) (12.0) mounted on the bearing component (13.0) so as to be pivotable about the pivot axis (S) for connection to the movable handle part (12), wherein the pivot bracket (12.0) has a pivot bearing device (4) for providing the pivot axis (S), and the bearing component (13.0) has at least one bearing device (4') which is coaxial with the pivot axis (S) and which is designed to cooperate with the pivot bearing device (4) for pivotably mounting the at least one pivot bracket (12.0) on at least one of the guide rails (13.1).

6. The coupling device (1) according to at least one of claims 1 to 5,
**characterised in that**
the sliding element (3) has a passage opening (3.5) connected to the guide receptacle (3.1), wherein the passage opening (3.5) overlaps the notch (2.1) of the coupling element (2), and wherein the coupling element (2) has, on an axis (X) parallel to the pivot axis (S), at least one outwardly projecting guide pin (2.2), preferably two outwardly projecting guide pins (2.2), and the sliding element (3) has at least one guide gap (3.6), preferably two guide gaps (3.6), which runs/run parallel to the coupling axis (K) and is/are connected to the guide receptacle (3.1), wherein the at least one guide pin (2.2) is arranged in the at least one guide gap (3.6).

7. The coupling device (1) according to claim 6,
**characterised in that**
the at least one guide pin (2.2) is designed to cooperate with the at least one pivot bracket (12.0) and projects with an end section out of the guide gap (3.6) of the sliding element (3), wherein the pivot bracket (12.0) has a guide groove (12.3) which runs in the radial direction towards the pivot bearing device (4) and in which the end section of the guide pin (2.2) is received.

8. The coupling device (1) according to at least one of claims 1 to 7,
**characterised in that**
the coupling device (1) has a spring element (5) which exerts a force in the direction of the coupling axis (K) on the coupling element (2) and holds the coupling element (2) in the engagement position.

9. The coupling device (1) according to claim 8,
**characterised in that**
the spring element (5) is arranged in a receiving recess (3.2) of the sliding element (3), which has the base (3.3) and adjoins the guide receptacle (3.1), forming a shoulder (3.4), and/or the coupling element (2) has a connecting piece (2.5) facing away from an opening side (2.7) of the recess (2.3) for engagement with the spring element (5).

10. The coupling device (1) according to at least one of claims 4 to 9,
**characterised in that**
the bearing component (13.0) has a block section (13.5) on which the guide rails (13.1) are arranged, wherein the block section (13.5) has a through-opening (13.4) along the longitudinal axis (L) for the force transmission element (22, 22').

11. A handle device (10) for a surgical instrument (100) for arrangement at a proximal end of a shaft (20), through which extends a force transmission element (21, 21') which defines a longitudinal axis (L) and has a stepped connecting element (22, 22') at a proximal end, wherein the handle device (10) has a handle part (12) movable about a pivot axis (S) and a coupling device (1) for connecting the movable handle part (12) to the force transmission element (21, 21'),
**characterised in that**
the coupling device (1) is a coupling device (1) according to at least one of claims 1 to 10.

12. A surgical instrument (100) having a shaft (20) through which extends a force transmission element (21, 21') movable along a longitudinal axis (L) with a stepped connecting element (22, 22') at a proximal end, wherein the surgical instrument (100) has at a proximal end of the shaft (20) a handle device (10) with a handle part (12) which is movable about a pivot axis (S) running perpendicularly to the longitudinal axis (L), and at a distal end of the shaft (20) has a tool (30) which is operatively connected to the force transmission element (21, 21'), and wherein the surgical instrument (100) has a coupling device (1) for connecting the movable handle part (12) to the force transmission element (21, 21'),
**characterised in that**
the coupling device (1) is a coupling device (1) according to at least one of claims 1 to 10.

13. A method for assembling a surgical instrument (100) comprising a handle device (10) and a force transmission element (21, 21') with a stepped connecting element (22, 22') at a proximal end using a coupling device (1) according to at least one of claims 1 to 10,
comprising the steps
- Arranging the coupling element (2) in the guide receptacle (3.1) of the sliding element (3) along the coupling axis (K) in the release position in which, at the height of the longitudinal axis (L), the width (b_{E}) of the notch (2.1) is greater than the width (bv) of the connecting element (22, 22');
- Inserting the connecting element (22, 22') at the proximal end of the force transmission element (21, 21') along the longitudinal axis (L) into the coupling device (1) through the notch (2.1) into the recess (2.3);
- Transferring the coupling element (2) into the engagement position in which, at the height of the longitudinal axis (L), the cross-sectional dimension (q) of the recess (2.3) in the direction of the longitudinal axis (L) corresponds to the longitudinal sectional dimension (1) of the connecting element (22, 22') and the width (b_{E}) of the notch (2.1) is smaller than the width (bv) of the connecting element (22, 22') such that the connecting element (22, 22') engages behind the notch (2.1) and comes to rest in the recess (2.3).

14. The method according to claim 13, wherein the force transmission element (21, 21'), which is designed to transmit a predetermined limit force, is selected according to a predetermined maximum limit force for a tool (30) arranged at a distal end of the force transmission element (21, 21').

15. A method for disassembling a surgical instrument (100) comprising a handle device (10), and a force transmission element (21, 21') with a stepped connecting element (22, 22') at a proximal end using a coupling device (1) according to at least one of claims 1 to 10,
comprising the steps
- Transferring the coupling element (2) from the engagement position in which, at the height of the longitudinal axis (L), the cross-sectional dimension (q) of the recess (2.3) in the direction of the longitudinal axis (L) corresponds to the longitudinal sectional dimension (1) of the connecting element (22, 22') and the width (b_{E}) of the notch (2.1) is smaller than the width (bv) of the connecting element (22, 22'), into the release position in which, at the height of the longitudinal axis (L), the width (b_{E}) of the notch (2.1) is greater than the width (bv) of the connecting element (22, 22'); and
- Removing the connecting element (22, 22') from the recess (2.3) of the coupling element (2) through the notch (2.1) by pulling out the force transmission element (21, 21') along the longitudinal axis (L).

## Revendications

1. Dispositif de couplage (1) pour un instrument chirurgical (100), qui présente un élément de transmission de force (21, 21'), qui définit un axe longitudinal (L) et présente un élément de liaison (22, 22') décalé à une extrémité proximale, et un dispositif de poignée (10) comportant une partie de poignée (12), qui est mobile autour d'un axe de pivotement (S) perpendiculaire à l'axe longitudinal (L), dans lequel le dispositif de couplage (1) pour relier l'élément de transmission de force (21, 21') à la partie de poignée (12) mobile présente un élément de couplage (2) comportant un évidement (2.3) pour loger l'élément de liaison (22, 22') et un élément coulissant (3) coopérant avec l'élément de couplage (2), qui est conçu pour coopérer avec la partie de poignée (12) mobile et pour se déplacer dans la direction de l'axe longitudinal (L),
**caractérisé en ce que**
l'élément de couplage (2) est logé de manière mobile dans un logement de guidage (3.1) de l'élément coulissant (3) le long d'un axe de couplage (K) qui court de manière perpendiculaire à l'axe longitudinal (L),
l'évidement (2.3) dans l'élément de couplage (2) est formé en correspondance avec l'axe de couplage (K) et présente une section transversale qui se rétrécit en partant dans la direction de l'axe de couplage (K), et
l'élément de couplage (2) présente une encoche (2.1) qui est reliée à l'évidement (2.3), dans lequel une largeur (b_{E}) de l'encoche (2.1) se rétrécit dans une direction parallèle à l'axe de couplage (K), et
dans lequel l'élément de couplage (2) est mobile le long de l'axe de couplage (K) entre
- au moins une position de libération, dans laquelle, à une hauteur de l'axe longitudinal (L), la largeur (b_{E}) de l'encoche (2.1) est supérieure à une largeur (bv) de l'élément de liaison (22, 22'), et
- au moins une position de mise en prise, dans laquelle, à la hauteur de l'axe longitudinal (L), la dimension de section transversale (q) de l'évidement (2.3) dans la direction de l'axe longitudinal (L) correspond à une dimension de section longitudinale (1) de l'élément de liaison (22, 22') et la largeur (b_{E}) de l'encoche (2.1) est inférieure à une largeur (bv) de l'élément de liaison (22, 22'), de sorte que l'élément de liaison (22, 22') vient en prise derrière l'encoche (2.1) et vient s'appuyer dans l'évidement (2.3).

2. Dispositif de couplage (1) selon la revendication 1,
**caractérisé en ce que**
une forme de section transversale de l'évidement (2.3) dans l'élément de couplage (2) perpendiculaire à l'axe de couplage (K) correspond à une forme d'une surface de section longitudinale de l'élément de liaison (22, 22') perpendiculaire à l'axe de couplage (K), dans lequel de préférence l'évidement (2.3) dans l'élément de couplage (2) est conçu de forme conique ou tronconique avec une section transversale circulaire et l'élément de liaison (22, 22') est conçu de forme sphérique, dans lequel la dimension de section transversale (q) de l'évidement (2.3) dans la direction de l'axe longitudinal (L) est un diamètre de la section transversale circulaire et la dimension de section longitudinale (1) de l'élément de liaison (22, 22') est un diamètre de l'élément de liaison (22, 22') de forme sphérique.

3. Dispositif de couplage (1) selon la revendication 1 ou 2,
**caractérisé en ce que**
le dispositif de couplage (1) peut être mis en prise avec au moins un premier élément de transmission de force (21) ou avec un second élément de transmission de force (21'), dans lequel le premier élément de transmission de force (21) présente un premier élément de liaison (22) décalé pour transmettre une première force limite et le second élément de transmission de force (21') présente un second élément de liaison (22') décalé pour transmettre une seconde force limite, qui est supérieure à la première force limite, dans lequel la dimension de section longitudinale (1) du second élément de liaison (22') est supérieure à la dimension de section longitudinale (1) du premier élément de liaison (22) et la largeur (bv) du second élément de liaison (22') est supérieure à la largeur (bv) du premier élément de liaison (22), et dans lequel l'élément de couplage (2) pour la mise en prise
- est agencé avec le premier élément de transmission de force (21) le long de l'axe de couplage (K) dans une première position de mise en prise dans laquelle, au niveau de la hauteur de l'axe longitudinal (L), la dimension de section transversale (q) de l'évidement (2.3) dans la direction de l'axe longitudinal (L) correspond à la dimension de section longitudinale (1) du premier élément de liaison (22) et la largeur (b_{E}) de l'encoche (2.1) est inférieure à la largeur (bv) du premier élément de liaison (22), de sorte que le premier élément de liaison (22) vient en prise derrière l'encoche (2.1) et vient s'appuyer sur l'encoche (2.3), et
- est agencé avec le second élément de transmission de force (21') le long de l'axe de couplage (K) dans une seconde position de mise en prise dans laquelle, au niveau de la hauteur de l'axe longitudinal (L), la dimension de section transversale (q) de l'évidement (2.3) dans la direction de l'axe longitudinal (L) correspond à la dimension de section longitudinale (1) du second élément de liaison (22') et la largeur (b_{E}) de l'encoche (2.1) est inférieure à la largeur (bv) du premier élément de liaison (22'), de sorte que le second élément de liaison (22') vient en prise derrière l'encoche (2.1) et vient s'appuyer dans l'évidement (2.3).

4. Dispositif de couplage (1) selon au moins l'une des revendications 1 à 3,
**caractérisé en ce que**
le dispositif de couplage (1) présente un composant de palier (13.0), qui est présent dans un boîtier (13) du dispositif de poignée (10), dans lequel le composant de palier (13.0) présente deux rails de guidage (13.1) qui s'étendent parallèlement à l'axe longitudinal (L), et dans lequel l'élément coulissant (3) présente sur un côté détourné du logement de guidage (3.1) un profil de guidage comportant un tourillon de guidage (3.7) conçu le long de l'axe de couplage (K), qui est reçu entre les deux rails de guidage (13.1).

5. Dispositif de couplage (1) selon la revendication 4,
**caractérisé en ce que**
le dispositif de couplage (1) présente au moins un, de préférence deux, étrier(s) pivotant(s) (12.0) monté(s) sur le composant de palier (13.0) de manière à pouvoir pivoter autour de l'axe de pivotement (S) pour la liaison avec la partie de poignée (12) mobile, dans lequel l'étrier pivotant (12.0) présente un dispositif de palier pivotant (4) pour fournir l'axe de pivotement (S), et le composant de palier (13.0) présente au moins un dispositif de palier (4') coaxial à l'axe de pivotement (S), qui est conçu pour coopérer avec le dispositif de palier pivotant (4) pour le montage pivotant de l'au moins un étrier pivotant (12.0) sur au moins l'un des rails de guidage (13.1).

6. Dispositif de couplage (1) selon au moins l'une des revendications 1 à 5,
**caractérisé en ce que**
l'élément coulissant (3) présente une ouverture de passage (3.5) reliée au logement de guidage (3.1), dans lequel l'ouverture de passage (3.5) recouvre l'encoche (2.1) de l'élément de couplage (2), et dans lequel l'élément de couplage (2) présente sur un axe (X) parallèle à l'axe de pivotement (S) au moins une broche de guidage (2.2) faisant saillie vers l'extérieur, de préférence deux broches de guidage (2.2) faisant saillie vers l'extérieur, et l'élément coulissant (3) présente au moins une fente de guidage (3.6), de préférence deux fentes de guidage (3.6), qui court/courent parallèlement à l'axe de couplage (K) et qui est/sont reliée(s) au logement de guidage (3.1), dans lequel l'au moins une broche de guidage (2.2) est agencée dans l'au moins une fente de guidage (3.6).

7. Dispositif de couplage (1) selon la revendication 6,
**caractérisé en ce que**
l'au moins une broche de guidage (2.2) est conçue pour coopérer avec l'au moins un étrier pivotant (12.0) et fait saillie avec une section d'extrémité hors de la fente de guidage (3.6) de l'élément coulissant (3), dans lequel l'étrier pivotant (12.0) présente une rainure de guidage (12.3) qui court dans la direction radiale du dispositif de palier pivotant (4) et dans laquelle est logée la section d'extrémité de la broche de guidage (2.2).

8. Dispositif de couplage (1) selon au moins l'une des revendications 1 à 7,
**caractérisé en ce que**
le dispositif de couplage (1) présente un élément de ressort (5) qui exerce une force dans la direction de l'axe de couplage (K) sur l'élément de couplage (2) et maintient l'élément de couplage (2) dans la position de mise en prise.

9. Dispositif de couplage (1) selon la revendication 8,
**caractérisé en ce que**
l'élément de ressort (5) est agencé dans un évidement de réception (3.2) de l'élément coulissant (3), qui présente le fond (3.3) et se raccorde au logement de guidage (3.1) en formant un épaulement (3.4),
et/ou l'élément de couplage (2) présente un embout (2.5) opposé à un côté d'ouverture (2.7) de l'évidement (2.3) pour la mise en prise avec l'élément de ressort (5).

10. Dispositif de couplage (1) selon au moins l'une des revendications 4 à 9,
**caractérisé en ce que**
le composant de palier (13.0) présente une section de bloc (13.5) sur laquelle sont agencés les rails de guidage (13.1), dans lequel la section de bloc (13.5) présente une ouverture de passage (13.4) le long de l'axe longitudinal (L) pour l'élément de transmission de force (22, 22').

11. Dispositif de poignée (10) pour un instrument chirurgical (100) destiné à être agencé à une extrémité proximale d'une tige (20) à travers laquelle s'étend un élément de transmission de force (21, 21'), qui définit un axe longitudinal (L) et présente à une extrémité proximale un élément de liaison (22, 22') décalé, dans lequel le dispositif de poignée (10) présente une partie de poignée (12) mobile autour d'un axe de pivotement (S) et un dispositif de couplage (1) pour relier la partie de préhension (12) mobile à l'élément de transmission de force (21, 21'), **caractérisé en ce que**
le dispositif de couplage (1) est un dispositif de couplage (1) selon au moins l'une des revendications 1 à 10.

12. Instrument chirurgical (100) qui présente une tige (20) à travers laquelle s'étend un élément de transmission de force (21, 21') mobile le long d'un axe longitudinal (L), comportant un élément de liaison (22, 22') décalé à une extrémité proximale, dans lequel l'instrument chirurgical (100) présente à une extrémité proximale de la tige (20) un dispositif de poignée (10) comportant une partie de poignée (12), qui est mobile autour d'un axe de pivotement (S) s'étendant perpendiculaire à l'axe longitudinal (L), et à une extrémité distale de la tige (20), un outil (30) qui est en liaison fonctionnelle avec l'élément de transmission de force (21, 21'), et dans lequel l'instrument chirurgical (100) présente un dispositif de couplage (1) pour relier la partie de poignée (12) mobile à l'élément de transmission de force (21, 21'),
**caractérisé en ce que**
le dispositif de couplage (1) est un dispositif de couplage (1) selon au moins l'une des revendications 1 à 10.

13. Procédé d'assemblage d'un instrument chirurgical (100) comportant un dispositif de poignée (10) et un élément de transmission de force (21, 21') comportant un élément de liaison (22, 22') décalé à une extrémité proximale, en utilisant un dispositif de couplage (1) selon au moins l'une des revendications 1 à 10,
comprenant les étapes
- d'agencement de l'élément de couplage (2) dans le logement de guidage (3.1) de l'élément coulissant (3) le long de l'axe de couplage (K) dans la position de libération dans laquelle, au niveau de la hauteur l'axe longitudinal (L), la largeur (b_{E}) de l'encoche (2.1) est supérieure à la largeur (bv) de l'élément de liaison (22, 22') ;
- d'insertion de l'élément de liaison (22, 22') à l'extrémité proximale de l'élément de transmission de force (21, 21') le long de l'axe longitudinal (L) dans le dispositif de couplage (1) à travers l'encoche (2.1) dans l'évidement (2.3) ;
- de transfert de l'élément de couplage (2) dans la position de mise en prise dans laquelle, au niveau de la hauteur de l'axe longitudinal (L), la dimension de section transversale (q) de l'évidement (2.3) dans la direction de l'axe longitudinal (L) correspond à la dimension de section longitudinale (1) de l'élément de liaison (22, 22') et la largeur (b_{E}) de l'encoche (2.1) est inférieure à la largeur (bv) de l'élément de liaison (22, 22'), de sorte que l'élément de liaison (22, 22') vient en prise derrière l'entaille (2.1) et vient s'appuyer dans l'évidement (2.3).

14. Procédé selon la revendication 13, dans lequel l'élément de transmission de force (21, 21') qui est conçu pour transmettre une force limite prédéterminée est sélectionné en fonction d'une force limite maximale prédéterminée pour un outil (30) qui est agencé à une extrémité distale de l'élément de transmission de force (21, 21').

15. Procédé de démontage d'un instrument chirurgical (100) comportant un dispositif de poignée (10), et un élément de transmission de force (21, 21') comportant un élément de liaison (22, 22') décalé à une extrémité proximale, en utilisant un dispositif de couplage (1) selon au moins l'une des revendications 1 à 10,
comprenant les étapes
- de transfert de l'élément de couplage (2) de la position de mise en prise, dans laquelle, au niveau de la hauteur de l'axe longitudinal (L), la dimension de section transversale (q) de l'évidement (2.3) dans la direction de l'axe longitudinal (L) correspond à la dimension de section longitudinale (1) de l'élément de liaison (22, 22') et la largeur (b_{E}) de l'encoche (2.1) est inférieure à la largeur (bv) de l'élément de liaison (22, 22'), à la position de libération dans laquelle, au niveau de la hauteur de l'axe longitudinal (L), la largeur (b_{E}) de l'encoche (2.1) est supérieure à la largeur (bv) de l'élément de liaison (22, 22') ; et
- de retrait de l'élément de liaison (22, 22') de l'évidement (2.3) de l'élément de couplage (2) à travers l'encoche (2.1) en tirant vers l'extérieur l'élément de transmission de force (21, 21') le long de l'axe longitudinal (L).
